# EUROPEAN PATENT APPLICATION

(11) **EP 3 373 008 A1**
(43) Date of publication of application: **12.09.2018**
(21) Application number: 18159989.5
(22) Date of filing: 05.03.2018
(51) Int. Cl.: G01N 33/564

(54) **IN VITRO METHOD AND KIT FOR PREDICTING CLINICAL OUTCOME IN PATIENTS WITH MEMBRANOUS NEPHROPATHY**

(30) Priority: 07.03.2017 IT 201700025190
(71) Applicant: PAD 4 DI EMANUELE GHIGGERI S.A.S., 16043 Chiavari Ge (IT)
(72) Inventor: Ghiggeri, Gian Marco, 16043 Chiavari GE (IT); Ghiggeri, Luca, 16043 Chiavari GE (IT)
(74) Representative: Di Giovine, Paolo

(57) **Abstract**

The present invention relates to an *in vitro* method for the prediction of the clinical outcome in terms of proteinuria and renal function of patients with Membranous Nephropathy. Prediction of such an outcome can be done by determining the levels in serum of anti-SOD 2 and/or anti-apha enolase IgG4 at the time of diagnosis of the renal lesion and before starting any therapy. An *in vitro* method for monitoring a therapy against membranous nephropathy in subjects affected by the disease that can be done by determining serum levels of anti-SOD2 and/or anti-alpha enolase IgG4 at the start and before the therapy and during the follow up. Positivity of one of the antibodies above recognizes among patients positive to anti-PLA2r epitopes the 100% of patients in which proteinuria will not remit after 12 months of therapies. This finding has an addictive and crucial potential for defining patients who can be treated.

## Description

### TECHNICAL FIELD OF THE INVENTION

The present invention is based on the characterization of nephrotoxic antibodies in patients with Membranous Nephropathy performed in the laboratories of the inventors (i.e. anti-SOD2 IgG4, anti-alpha enolase IgG4) and defines, for the first time, the possibility to predict the clinical outcome in the same patients simply on the basis of the presence of these antibodies in serum at the time of diagnosis of the disease and prior any therapeutic approach is started. Actually, the presence of the antibodies above is additive to other antibodies already described in Mambranous nephropathy (anti-PLA2R1, anti-PLA2R1 epitopes) that can predict to some extent the outcome; the concomitant presence of anti-PLA2R1 (global or epitopes) with anti-SOD2 and anti-alpha Enolase is able to define more than 95% of poor-outcomers. On such nosologic evolution it is proposed an *in vitro* method for the prediction of the clinical outcome in terms of proteinuria and renal function in patients with Membranous Nephropathy if utilized at the discovery of the disease; an *in vitro* method that is able to predict the clinical outcome in terms of proteinuria and renal function in patients with Membranous Nephropathy in response to therapies. With the term 'clinical response' it is here indicated the remission of proteinuria, complete or partial (i.e <0,3 gr day or 3,5 gr day) and normal renal function (CK-EPI>60 ml min⁻¹ 1,73 m²)after one year of follow up. A kit for the determination of anti-SOD2 and/or anti-alpha enolase antibodies of IgG4 isotype that allows the prediction at the start of the disease of the clinical outcome in terms of proteinuria and renal function during 1 to 3 years follow-up; a kit for the determination of anti-SOD2 and/or anti-alpha enolase antibodies of IgG4 isotype that allows the prediction of the clinical response to therapies in patients with Membranous Nephropathy.

### STATE OF PRIOR ART

Membranous Nephropathy (MN) is a clinical-pathological condition characterized by deposition of auto-antibodies in glomeruli that produce proteinuria and renal lesions and that may evolve to renal failure. Implication of an inflammatory cascade following antibody deposition involving free radicals and PKC have been proposed as an evolving mechanism. Therapies are now in progress and are designed to reduce antibody production and/or clinical signs of the disease that are mainly proteinuria and decline of renal function but chances are that blocking the post-antibody inflammatory cascade could be crucial to modify evolution. Biomarkers of the pathology could help to predict outcome and tailor the drug approach.

Idiopathic Membranous Nephropathy is the most common cause of nephrotic syndrome in adults {Makker, 2011 #81}. It is characterized by a well-defined pattern of sub-epithelial IgG4 deposition that constitutes the basis of the pathologic diagnosis. Membranous Nephropathy is considered the basic model for any autoimmune glomerulonephritis ("primary MN") or occurs as a manifestation of systemic conditions, including cancer, infections, reactivity to drugs or lupus erythematosus ("secondary MN"{Glassock, 2010 #80;Ronco, 2015 #460;Lefaucheur, 2006 #90}. It is of note that the natural history may vary in relation of unknown factors: about 25-30% of MN patients undergo spontaneous remission, others respond to treatments while 25% show progression to end stage renal disease in spite of any therapeutic approaches{Fervenza, 2008 #458;Polanco, 2010 #459}.

Formation of auto-antibodies directed against specific podocyte targets is considered the culprit of pathogenesis and their determination constitutes the basis for a new classification of potentially different forms of primary MN. High circulating levels of anti-human phospholipase A receptor (PLA2r) IgGs and their presence in renal tissues have been reported in a high proportion of MN patients, from 75% in Caucasians to 50% in Japanese populations{Beck, 2009 #119;Hoxha, 2012 #461;Kimura, 2016 #471}. Genetic studies support a causal role of anti-PLA2r in iMN based on an association of the disease with *6p21 HLA-DQA1* and *2q24 PLA2R* loci in both Caucasians{Stanescu, 2011 #193} and in Asians{Lv, 2013 #196}. Anti-Thrombospondin type-1 domain containing 7A (THSD7A) antibodies are rare, and so far reported to be positive in 5-10% of anti-PLA2r negative patients {Tomas, 2014 #124;Tomas, 2016 #126}. Auto-antibodies targeting other podocyte antigens, including superoxide dismutase (SOD2){Prunotto, 2010 #122}, aldose reductase (AR) and alpha enolase (eno){Bruschi, 2011 #70;Kimura, 2016 #471} have been discovered in our laboratory and described in association with anti-PLA2r{Murtas, 2012 #112} but their role remains to be elucidated.

Circulating antibodies in MN have been extensively investigated in the past however showing variable representation and ability to predict outcome. Ruggenenti{Ruggenenti, 2015 #443} in a prospective study showed the same outcomes in patients with and without detectable anti-hPLA2r IgGs antibodies at baseline; Bech{Bech, 2014 #446} and Hofstra {Hofstra, 2012 #447}showed the same percent of both complete and partial remission in patients with different tertiles of antibody titer, high levels only predicting spontaneous remission. Finally, a prospective multicenter study in Germany{Hoxha, 2014 #448} that considered only anti-hPLA2r patients showed a faster reduction of proteinuria in presence of anti-hPLA2r IgGs higher than the median (overall 133 patients were studied) and multivariate Cox analysis identified anti-hPLA2r levels as a risk factor for not achieving remission. In a retrospective study, Kanigicherla {Kanigicherla, 2013 #450} showed an association of high anti-hPLA2r with doubling of creatinine in the long-term. Finally, all studies confirmed the predictive role of anti-hPLA2r IgGs levels at the follow-up{Oh, 2013 #455;Bech, 2014 #446}. Therefore, serum levels of anti-PLA2r and anti-THSD7A antibodies are considered strong diagnostic biomarkers of MN{Ruggenenti, 2015 #443;Bech, 2014 #446;Hofstra, 2012 #447;Hoxha, 2014 #448} that means that their presence suggest the existence of renal lesions typical of MN but they probably do not predict the clinical outcome of the diasese. Recent studies have also demonstrated the presence of circulating antibodies versus.{Seitz-Polski, 2015 #433;Kao, 2015 #436;Fresquet, 2015 #437;Seitz-Polski, 2016 #435} PLA2r epitopes that would vary in relation of conformational changes of the molecule and the presence of selective antibodies in the circulation may be the result of instability of the PLA2r molecule. The presence of anti-epitopes in circulation is associated with poor outcome in terms of proteinuria allowing to identify a part of these patients. Only few studies focused the meaning of circulating IgG4 versus superoxide dismutase (SOD2){Prunotto, 2010 #122}, aldose reductase (AR) and alpha enolase (eno){Bruschi, 2011 #70;Kimura, 2016 #471} in MN patients. In view of the reported implication of oxidants in the post-antibody deposition of anti-PIA2r antibodies the rise of anti-oxidant enzymes such as SOD2 could determine a sort of balance and reduce the oxidative insult in glomeruli and limit the inflammatory cascade. This is pertinent to the reported finding by our research group of the presence of anti-SOD2 antibody in patients with MN in view of the possibility that anti-SOD2 IgG4 could block the anti-oxidant activity of SOD2. In this view, anti-SOD2 antibodies would play a negative role on the balance between first deposition of ant-PLA2r antibodies, the induction of an inflammatory phase (oxidant production) and the anti-inflammatory step represented by SOD2 production. On the same way, the presence of anti-alpha enolase antibodies would block the transformation of plasminogen transform into plasmin, that is the active molecule for proteolysis since it is known that plasmin degrades extracellular matrix components and activates, in parallel, other metalloproeases. We lack studies that match all the pathogenetic phases above and focus on potential interconnection between presence of anti-SOD2 IgG4, anti-alpha Enolase IgG4 and anti-PLA2r epitope antibodies as modifiers of clinical outcome of MN.

The need of identifying specific markers of MN progression in man and developing not invasive diagnostic/prognostic instruments allowing a possibly early identification of progressors is shared by the scientific community in order to define a suitable and timely therapeutic strategy. The use thereof in the therapeutic follow up would provide a crucial help to the clinician in the attempt of modulating a therapy which usually is long and provides several therapeutic associations involving drugs with high toxicity

### SUMMARY OF THE INVENTION

The present invention relates to an *in vitro* method based upon determining circulating antibodies able to predict the clinical outcome and monitor patients with Membranous Nephropathy (MN). With the term 'clinical outcome' it is here indicated the remission of proteinuria (partial or complete) and maintenance of normal renal function in patients with Membranous Nephropathy after one year of follow up. The second improvement given by the present invention is related to the possibility to predict the clinical response to therapies that are classically utilized, with variable results, in patients with MN. The search starting objective which brought to the discovery that is topics of the present patent was the characterization of the target antigens existing in the kidney, seat of the inflammatory lesion, that characterize MN. Based upon the results of the renal study which brought to the identification of specific auto-antibodies versus endogenous proteins, then one reached the determination of the same antibodies in the serum and the demonstration that high circulating levels are associated with the presence of MN. Such research was carried out for the first time on man by using renal tissues directly obtained by means of *in vivo* organ biopsy. The conclusions deriving therefrom then are based upon studies which were for the first time carried out on human renal tissue and had been made possible by the development of the proteomic survey techniques (laser capture, mass spectrometries, two-dimensional electrophoresis, etc.).

The present invention is based upon the finding that subjects affected by MN, and wherein alterations of the renal structure and function typical of MN are observed, have higher renal and circulating levels than normal controls of some autoantibodies of the IgG4 isotype, antibodies having as antigenic target endogenous proteins expressed by the kidney such as SOD2 and/or α-enolase. The demonstration of the presence in the renal glomerulus of antibodies of IgG4 class versus the above proteins (that is SOD2 and/or α-enolase), carried out for the first time by the inventors represented the basis of the herein-described application. The identification of new target proteins of nephrotoxicity (above all anti-SOD2 and/or anti-aenolase) antibodies of IgG4 allowed evolutions both on the mechanisms involved in the renal damage and on the possibility of evaluating directly the renal and circulating levels of such specific antibodies as disease markers. In fact, herein it is demonstrated that patients affected by MN and that have high concentrations of circulating anti-SOD2 and/or anti-aenolase IgG4 at the time when the disease was diagnosed have a poor clinical outcome in terms of persistence of proteinuria and worsening of renal function. The same patients have a high probability of no response to therapies. The presence of anti-SOD2 in serum of patients with MN is also additive to the presence of other antibodies that are typically high in MN, such as anti-PLA2r IgGs and are, in particular, additive to the presence of anti-epitope antibodies. Then, it is important noting that there is a multiple composition of the autoantibodies involved in MN but it is herein demonstrated that such promiscuity is very selective and it has as target only some endogenous renal proteins and/or antigens planted therein and furthermore it involves in specific way antibodies of IgG4 isotype. As demonstrated in the herebelow section "Materials and Methods", higher levels of IgG4 versus SOD2 and/or αenolase is associated with a high risk of no clinical response after oneyear proteinuria in patients with MN. Moreover, it is here also demonstrated that the presence of anti-SOD2 and anti-alpha Enolase antibodies has an addictive predictive value in those patients who are positive to anti-PLA2r epitope antibodies allowing the identification of almost the 100% of 'poor outcomers' in terms of proteinuria

In particular, anti-SOD2 and/or anti-aenolase IgG4 antibodies can be advantageously used as markers of outcome in patients with MN. In fact, the determination of serum levels of anti-SOD2 and/or anti-aenolase IgG4 thereof as markers it is here demonstrated is a highly sensible and specific instrument for MN since they result absent in samples of subjects affected by nephritis with different aetiology. It appears clear that one of the advantages associated to the herein-described invention consists in the possibility of obtaining a prediction of clinical outcome of MN in advance with respect to typical symptoms of this pathology. A particularly advantageous aspect of the present invention is that anti-SOD and/or anti-aenolase IgG4 antibodies can be dosed even in a blood or serum sample without the need then of intervening in invasive way on the subject by means of a renal tissue collection by biopsy.

Therefore, it is herein described for the first time a specific, sensible and a little invasive *in vitro* method for predicting and monitoring in therapeutic follow up renal lesions in subjects affected by MN.
Therefore a first subject of the present application is formed by:
- an *in vitro* method for predicting and monitoring during follow up the outcome in a subject affected by MN and in particular the clinical response that is mainly normalization of proteinuria and renal function comprising the following steps:
   a) determining the concentration of IgG4 antibodies versus at least one of the antigens selected from the group: SOD2 and/or αenolase in a biological sample of said subject;
   b) comparing said concentration in said biological sample with a control value wherein an increase in the concentration of said antibodies with respect to said control value indicates a high risk of no achieving a clinical improvement of proteinuria after 1 year follow-up.

It is also a subject of the present invention a kit for predicting and/or for monitoring a therapy against MN in subjects suffering from MN comprising:
- at least an aliquot of one or more reagents for the determination of IgG4 antibodies versus at least one of the antigens selected from the group: SOD2 and αenolase in a biological sample of said subject and
- at least an aliquot of one positive control comprising IgG4 versus at least one of the antigens selected from the group: SOD2 and αenolase.

In particular the method comprises the following steps:
a) determining the levels of auto-antibodies of IgG4 isotype versus all target antigens from the group: SOD2 and/or αenolase, in a biological sample of said subject and
b) comparing the levels of the above auto-antibodies with those obtained starting form a control sample,
wherein an increase in the levels of said auto-antibodies with respect to said control levels indicates the presence of MN in the subject.

Additional advantages, as well as the features and the use modes of the present invention will result evident from the following detailed description of some preferred embodiments, shown by purely way of example and not with limitative purpose.

### DESCRIPTION OF THE FIGURES

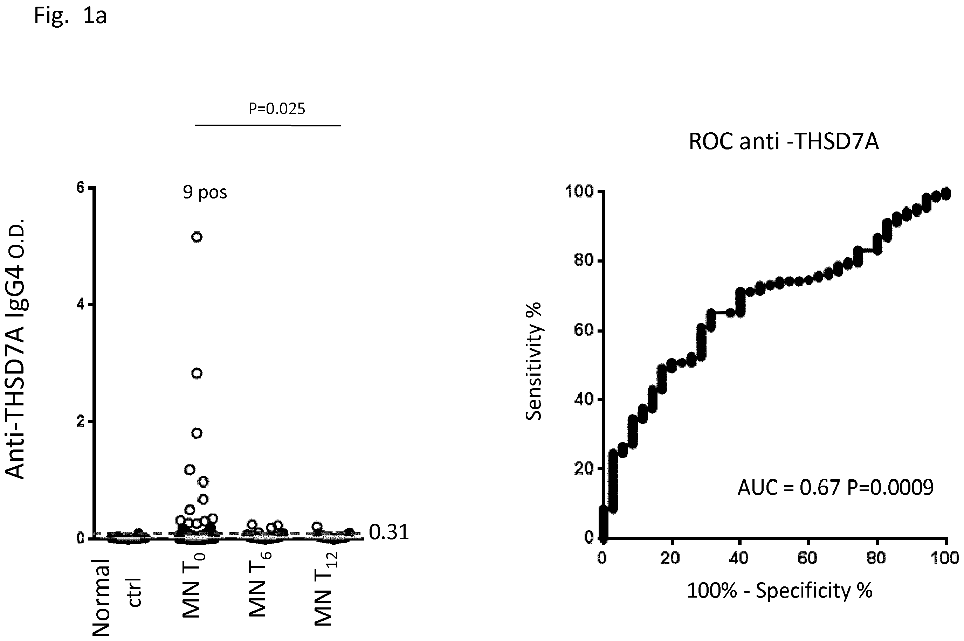

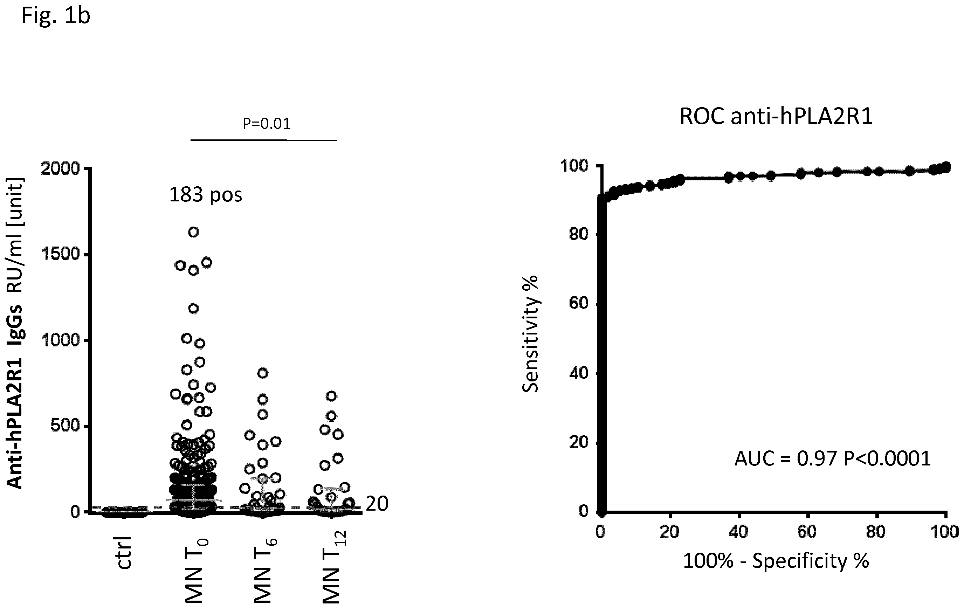

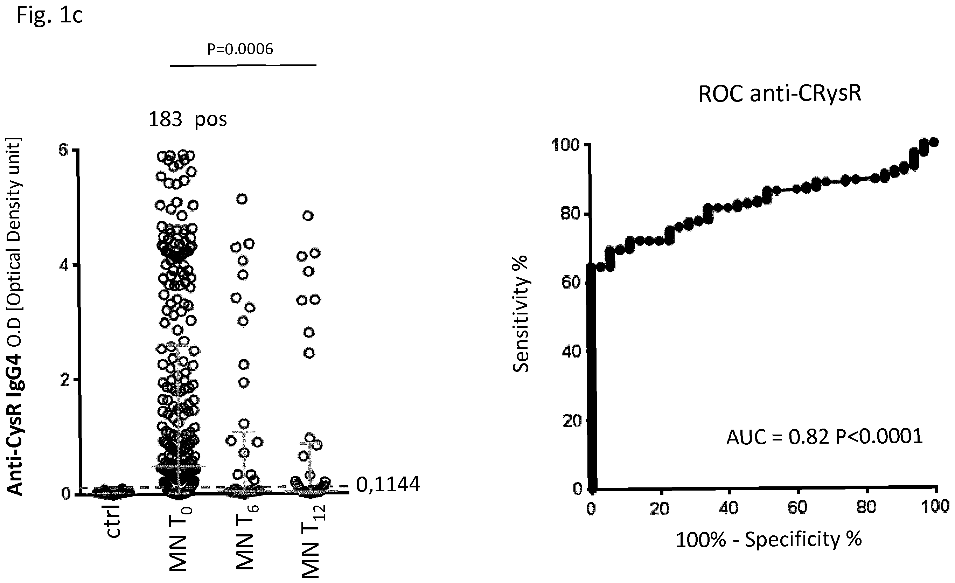

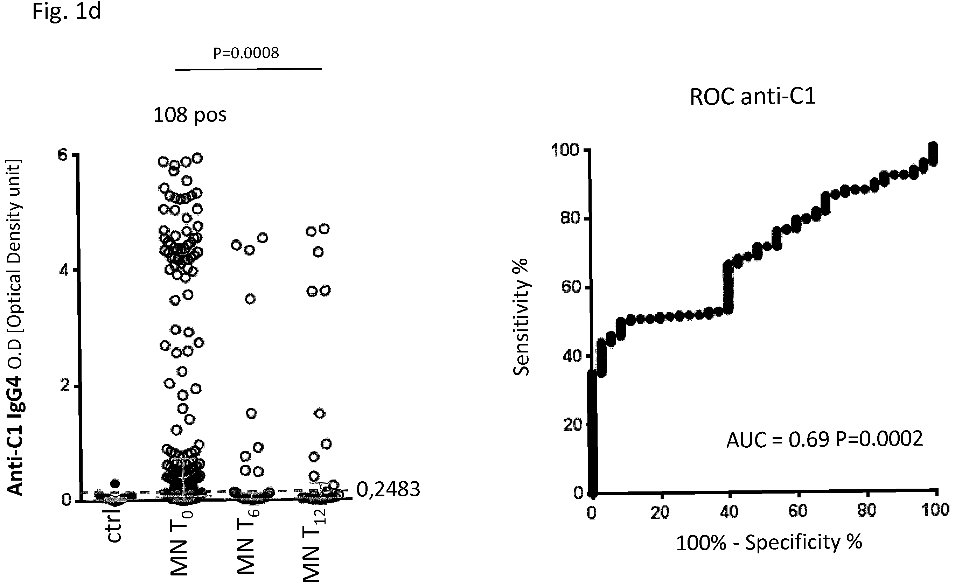

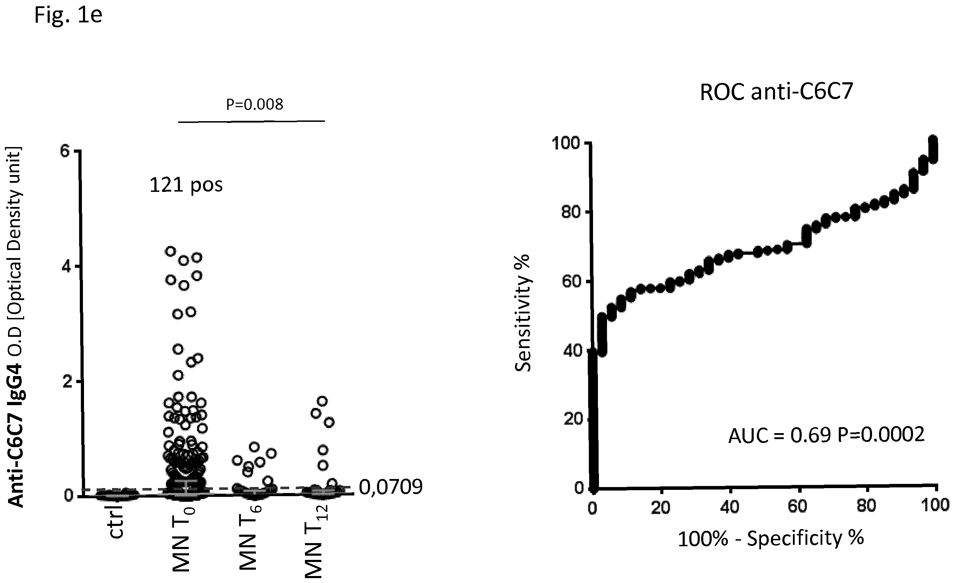

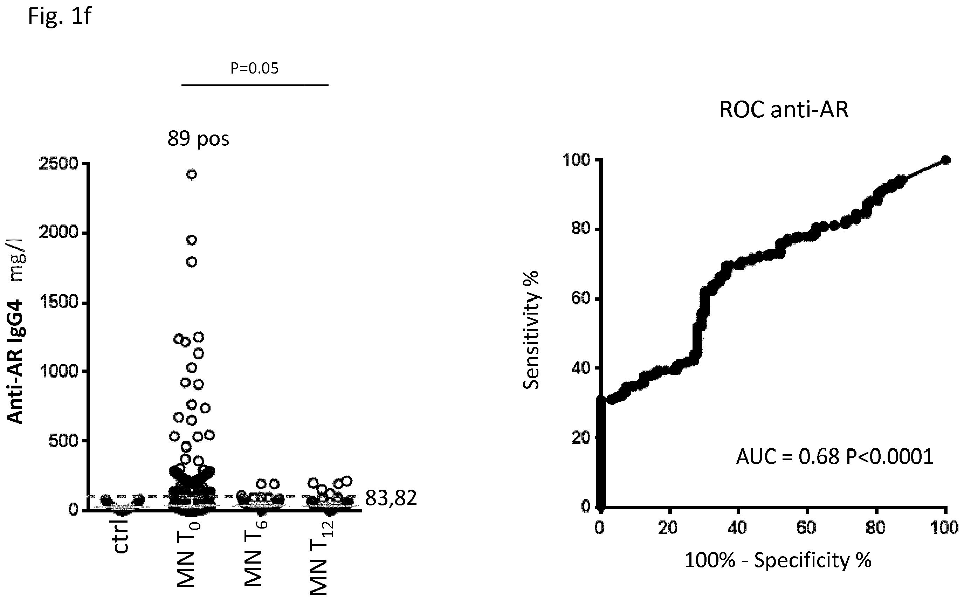

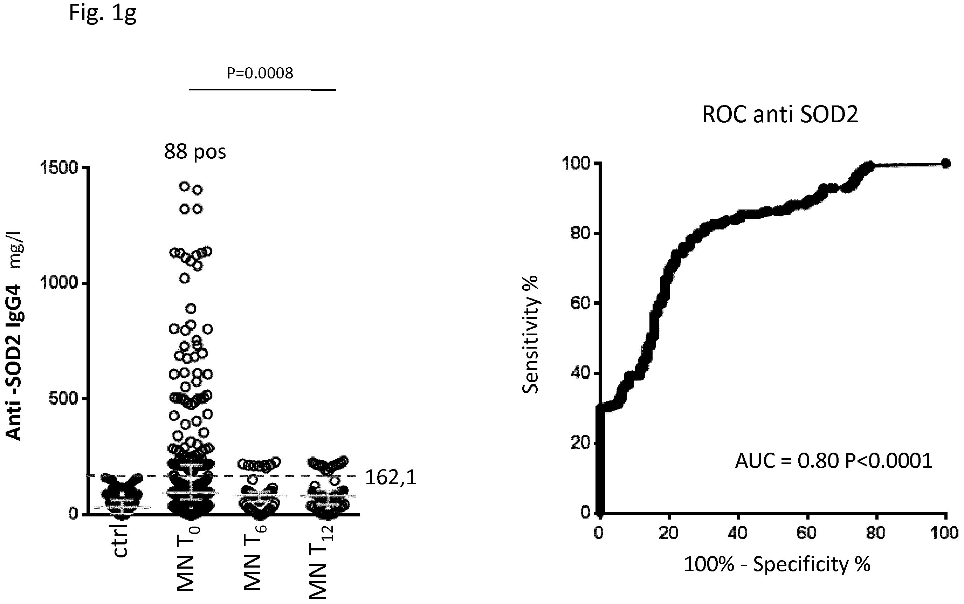

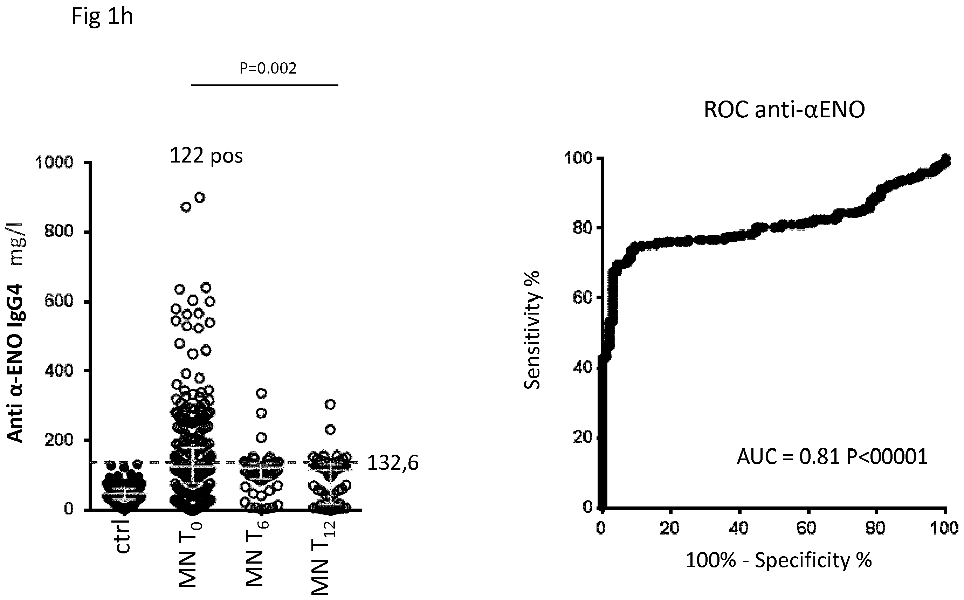

**Figure1****. Circulating antibodies.** Serum antibodies versus the whole panel of membrane and anti cytoplasm antigens: anti-THSD7A r **(a),** (IgG4) against circulating humanPLA2 and its epitopes (anti-CysR, anti-C1, anti-C7)**(b,c,d,e)** were determined in a large cohort of patients with Membranous Nephropathy. The same cohort was tested for anti-AR **(f),** anti-SOD2 **(g)** and anti-alpha enolase **(h)** IgG4. In the case of anti-THSD7A antibody positivity was tested by matching ELISA, immunofluorescence and western-blot and only multiple positivity were considered. The level of each antibody was evaluated at the time of diagnosis (To) and after 6 and 12 months (T₆, T₁₂) during which the majority of patients received at least one drug; only a part of patients remained untreated for the whole period. Specificity and sensitivity of each antibody versus normal controls were calculated with ROC curves. For anti human PLA2r IgGs results are given as RU/ml while for anti-SOD2, AR, anti-alpha enolase results are given as mg/ml. For antibodies versus epitopes results were given as chemiluminescence OD arbitrary units that corresponds to one unit of signal intensity of chemiluminescence detected by VersaDoc and computed with QuantyOne software (Bio-Rad). The red dash-line indicates the limit of positivity (i.e. values that minimize the geometric distance from 100% sensitivity and 100% specificity on the ROC curves) that were calculated for each antibody; continuous lines indicate instead the inter-quartile ranges

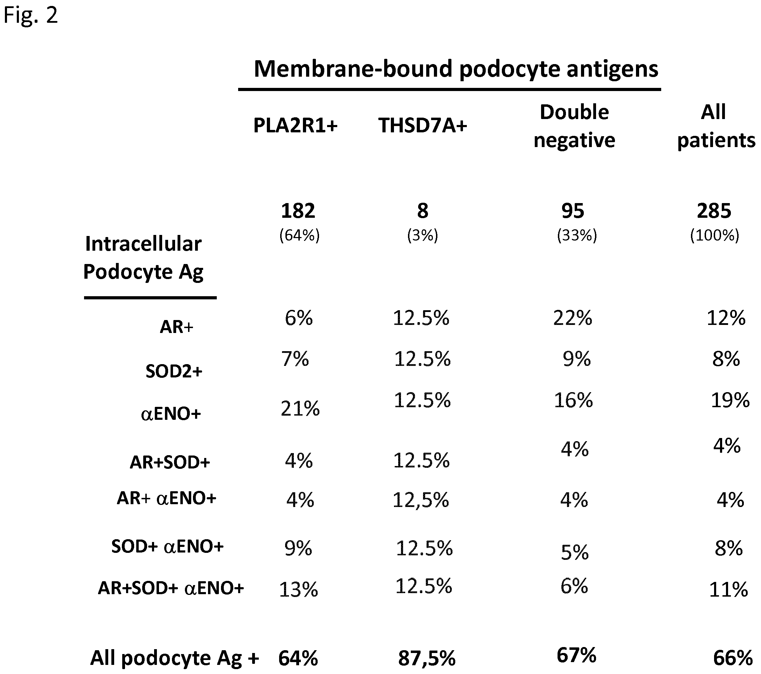

**Figure 2****. Distribution of circulating antibodies against membrane-bound and intracellular antigens in the cohort of iMN patients.** Patients were sub-divided according to the presence of circulating antibodies versus the membrane-bound podocyte antigens PLA2R1 or THSD7A; patients negative for the two antibodies were defined as Double Negative. The three groups of patients so categorized (i.e. PLA2Ra+, THSD7A+ and Double negative) were further sub-divided according to single positivity for each antibody towards the three intracellular podocyte antigens (AR, SOD2, αENO) and/or for their composite positivity (AR+SOD+; AR+ αENO+; SOD+ αENO+; AR+SOD+ αENO +). For normal values of anti-hPLA2R1 IgGs it was utilized the limit of 20 RU /ml {Dahnrich, 2013 #481}; normal limits for other antibodies were calculated from ROC curves (Supplement Figure 2a-2b and 2f-2h) in which case the cut off was represented by the value that minimized the geometric distance from 100% sensitivity and 100% specificity on the ROC curves{Zweig, 1993 #476;Zweig, 1993 #477}.

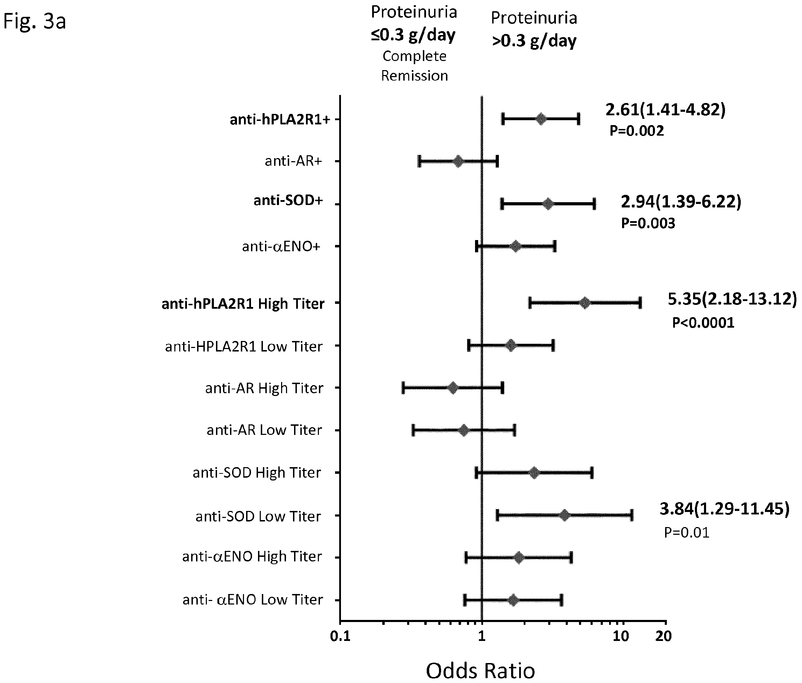

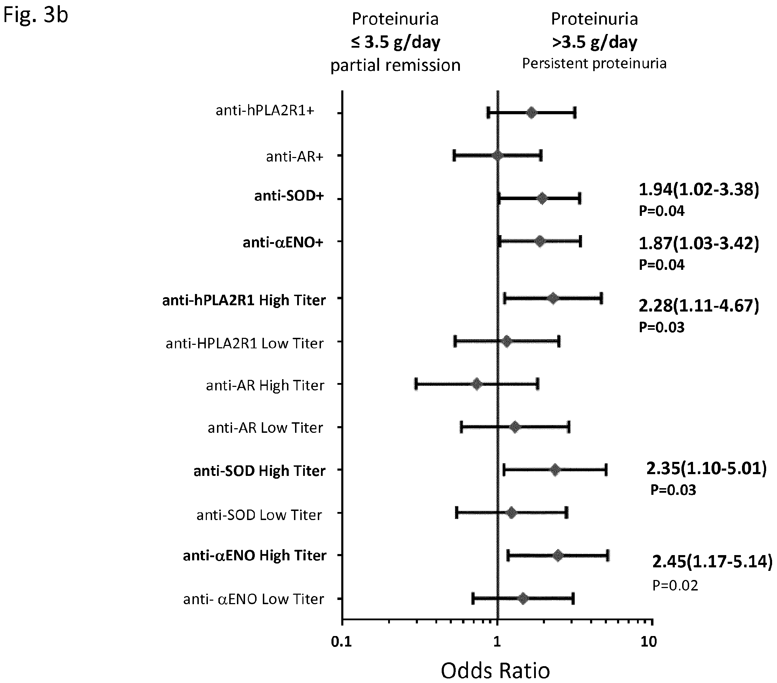

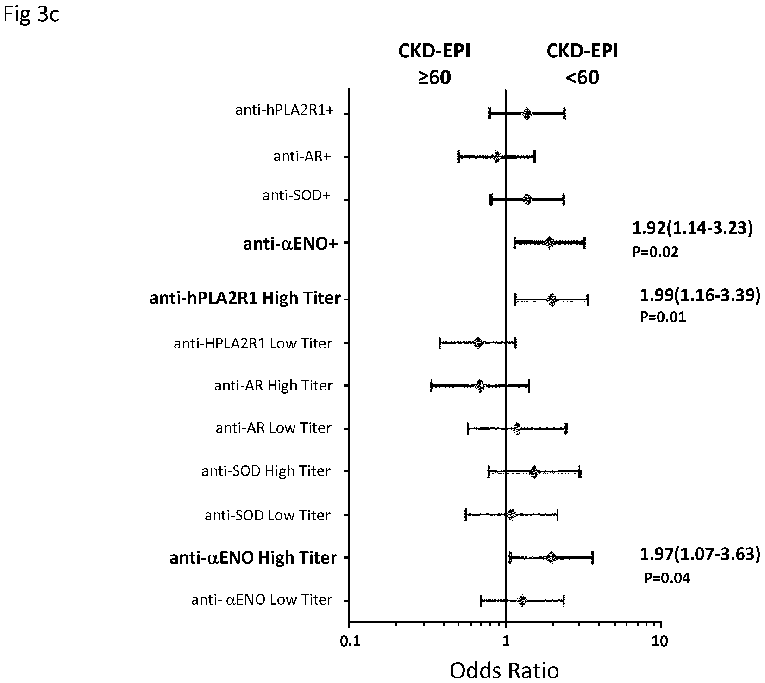

**Figure 3****. Risk assessment for proteinuria.** The risk of maintaining proteinuria after 12 months of follow up (T₁₂) associated with serum levels of each antibody at To was calculated by contingence tables in which patients were stratified for having achieved complete remission of proteinuria (<0.3 gr 24 hours) or were still presenting persistent proteinuria **(a);** the risk of maintaining nephrotic proteinuria **(b)** (i.e proteinuria>3.5 gr day at T₁₂) was calculated considering patients as in 'a'. In the former case, positivity for circulating anti-hPLA2r IgGs and of anti-SOD2 IgG4 was associated with high risk and considering double positivity for anti-SOD2 and anti-hPLA2r conferred the highest risk of no achieving complete remission (OR 5.58) whereas no risk was associated with positivity of antibodies against hPLA2ralone. The same results (i.e. positivity to high levels of anti-PLA2r and of anti-SOD2 antibodies) were obtained from the analysis of the risk of maintaining nephrotic proteinuria (>3.5 gr); in this case the presence of αENO antibodies gave the highest rank (OD 2,45, CL 1,17-5,14). In **(c)** it is shown the risk of worsening renal function (CK-EPI < 60 ml/min/1,73 m2) associated with the presence of antibodies at To. In this case, anti- αENO emerged as the unique parameter with a statistical association

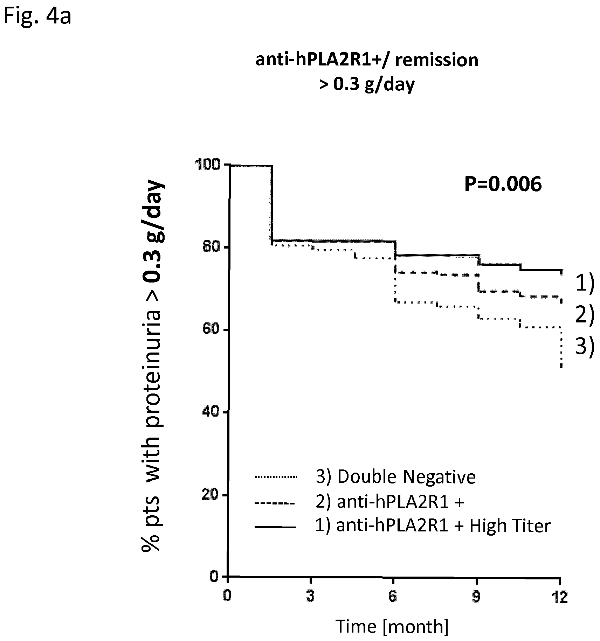

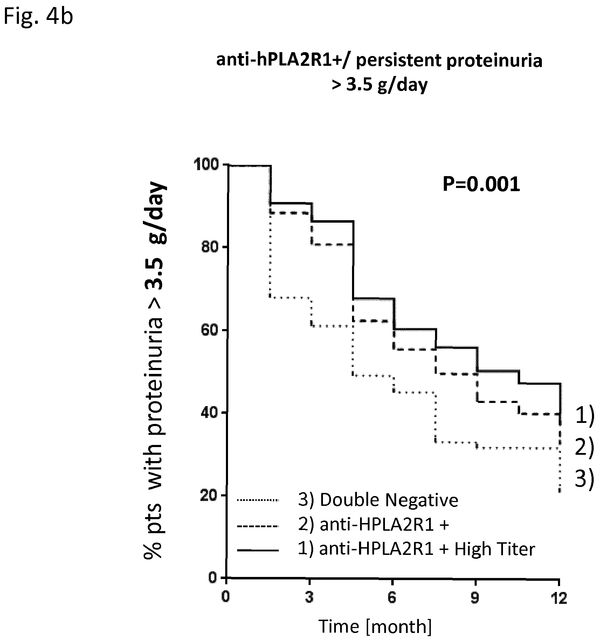

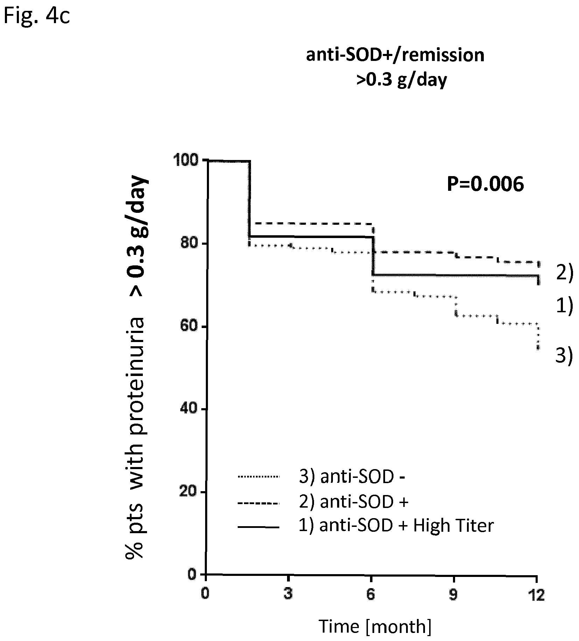

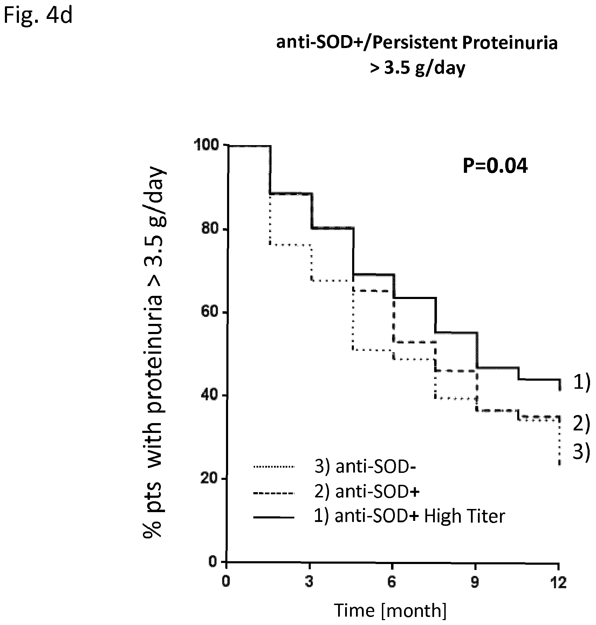

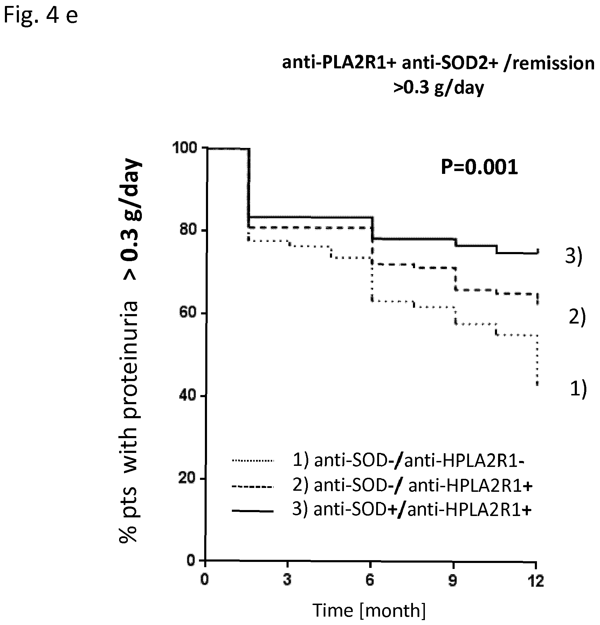

**Figure 4****. Survival for proteinuria.** Kaplan Meier analysis for oneyear outcome of proteinuria and presence of circulating antibodies. Patients were grouped as in Figure 3 and only results statistically significant were reported for different associations: **(a** and **b)** anti-hPLA2r IgGs; **(c** and **d)** anti-SOD2 IgG4; **(e)** anti-SOD2 associated with anti-hPLA2r. Mixed positivity of anti-SOD2 with anti-hPLA2r antibodies gave the most remarkable results, according to which the mixed presence of anti-SOD2 IgG4 with any of the above antibodies was associated with the poorest outcome.

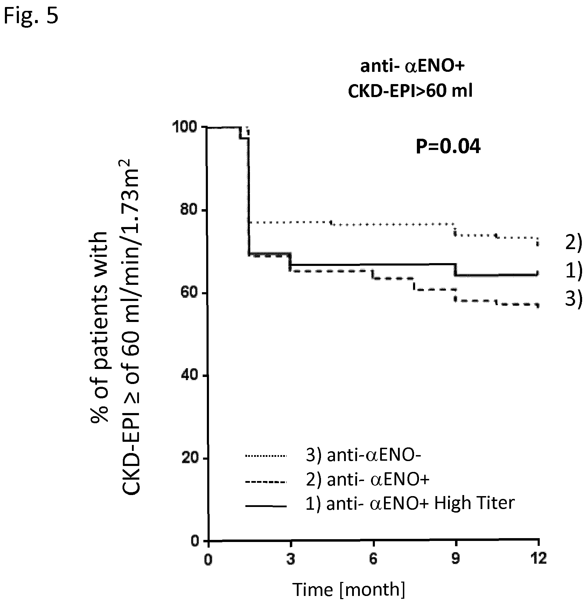

**Figure 5****. Risk assessment for renal function and survival.** The risk of reducing renal function after 12 months in patients with different levels of antibodies at To was calculated utilizing CKD-EPI <60 and <30 ml min 1.73² as limits. In the case of the first target (i.e. renal function >60 ml) the presence of anti-alpha enolase IgG4 emerged as good predictors of loosing renal function .

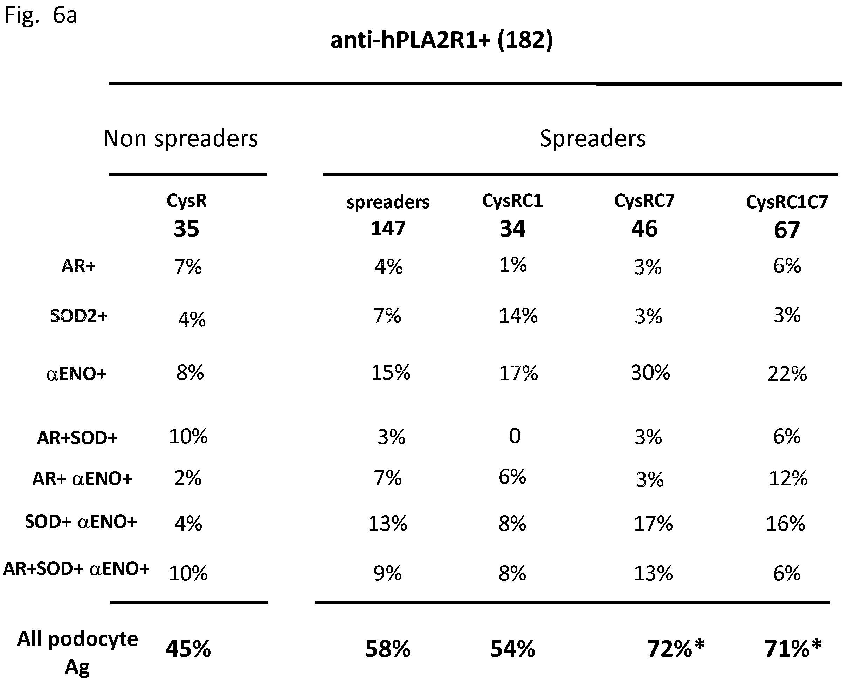

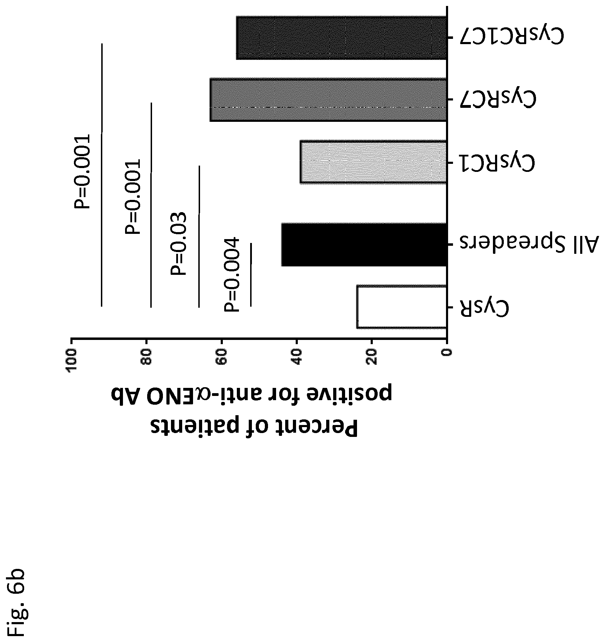

**Figure 6****. Distribution of circulating antibodies against anti-epitopes and intracellular antigens in the cohort of iMN patients. (a)** Patients positive for anti-PLA2R1 antibodies were split according to their PLA2R1 epitope profiles that identified "non spreaders" (CysR only) or "spreaders" (CysRC1, CysRC7 and CysRC1C7). Spreaders and non-spreaders were further split for the presence of single (anti-AR, anti-SOD2, anti-aENO) and composite anti-intra podocyte antigens antibodies (AR+SOD+; SOD+ αENO+; AR+SOD+ αENO+). For normal values of anti-hPLA2R1 IgGs it was utilized the limit of 20 RU /ml {Dahnrich, 2013 #481}; normal limits for other antibodies were calculated from ROC curves (Supplement Figure 2c-2e) in which case the cut off was represented by the value that minimized the geometric distance from 100% sensitivity and 100% specificity on the ROC curves.{Zweig, 1993 #476;Zweig, 1993 #477}. * indicates a significant difference
   **(b)** this graph shows that the percent of patients' positive for anti- αENO antibodies in each group of spreaders increases with the complexity of spreding and it was maximal in patients positive for anti-CysRC1C7.

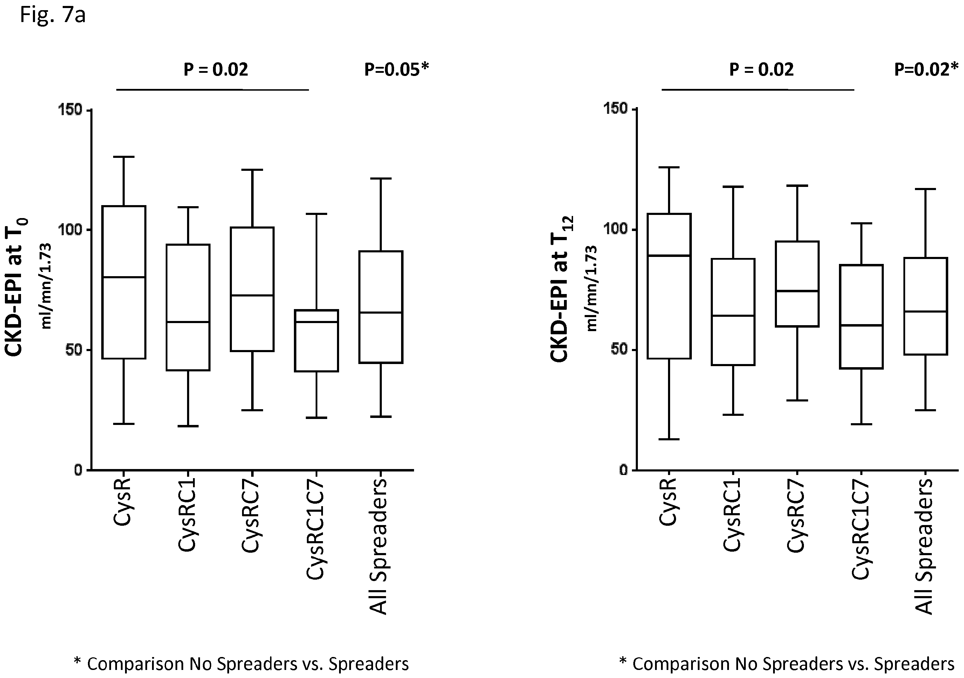

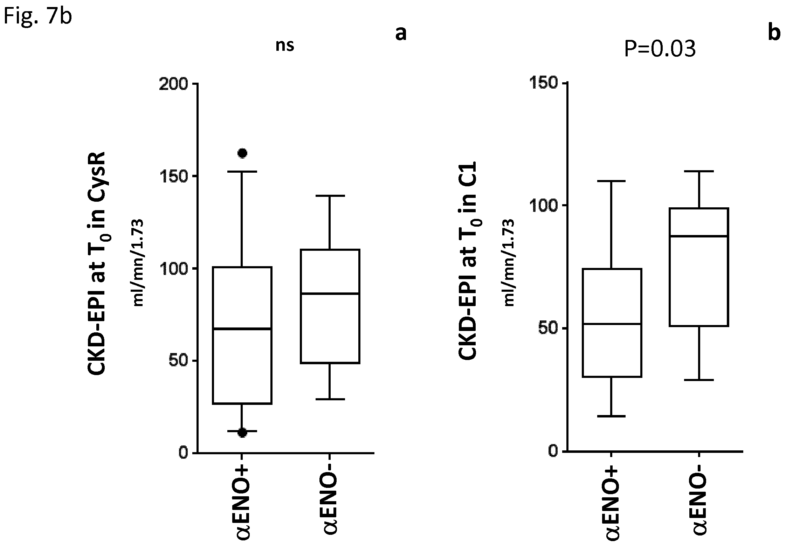

**Figure 7****. Proteinuria and renal function in patients positive to single antibodies. (a)** Renal function (CKD-EPI) at To and T₁₂ in patients positive for antibodies versus each epitopes of PLA2R1; **(b)** patients positive for different epitopes were split for the presence or absence of anti- αENO antibodies showing that this associationworsens renal function in comparison with anti-epitopes alone. Data are reported as median and interquartile range. Mann-Whitney or Kruskal-Wallis tests were used to compare the auto antibodies titer respectively for two or more of two un-pared samples. Two-tailed P-values ≤ 0.05 were considered significant. (ns; not significant)

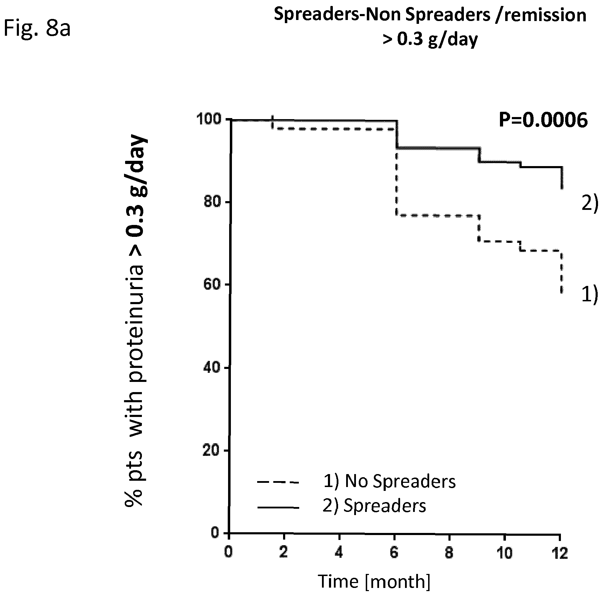

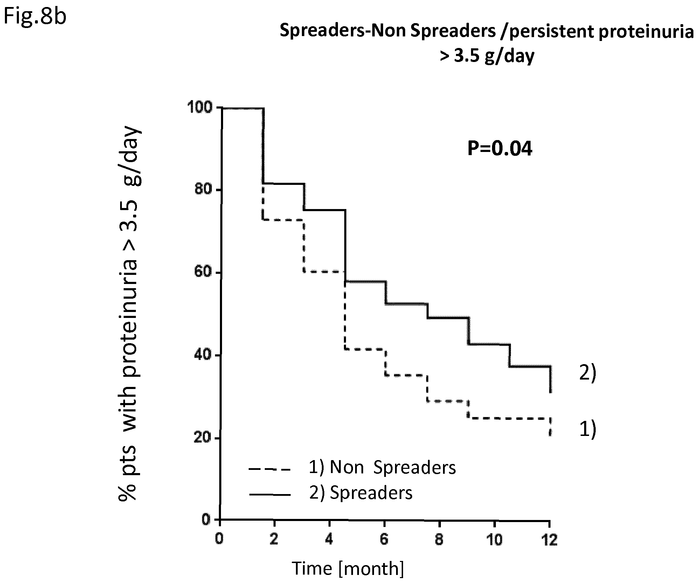

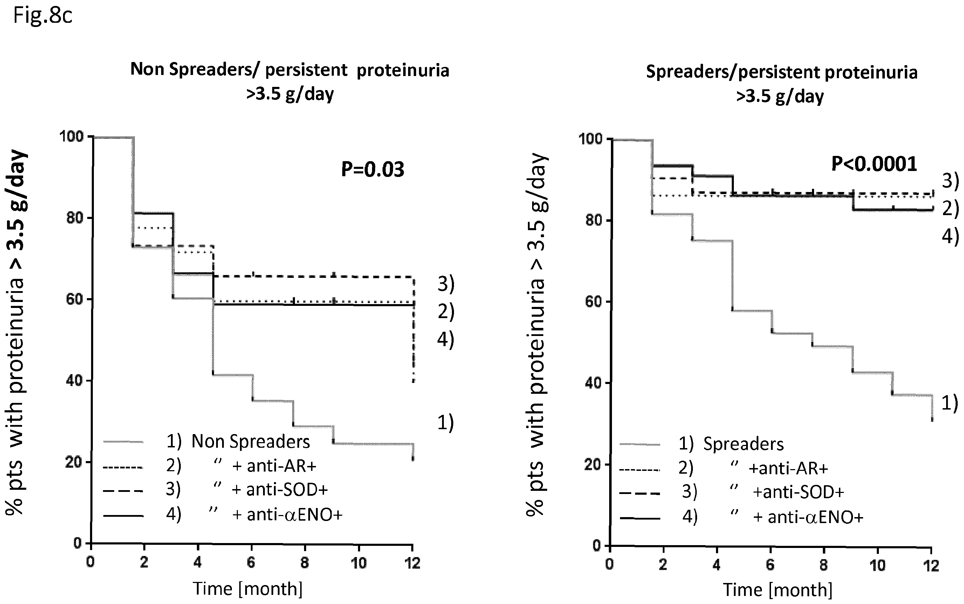

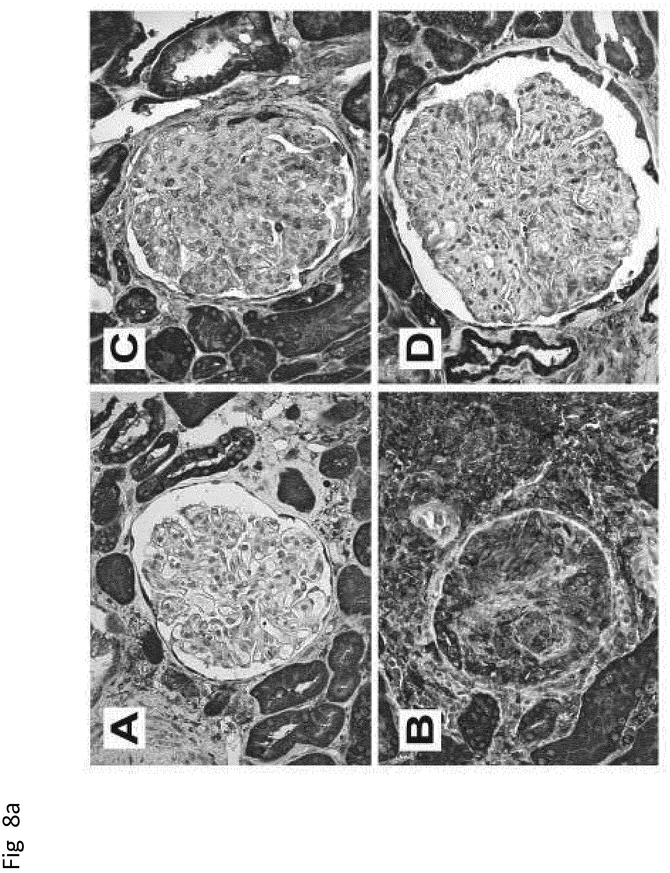

**Figure 8****. Survival of proteinuria is associated with multiple antibodies.** Kaplan Meier analysis was utilized to assess the one year outcome of proteinuria in patients subdivided for the presence of circulating antibodies. The targets were lack of remission of proteinuria (>0.3 gr/day) or maintenance of high proteinuria (>3.5 gr/day) after 12 months. **(a,b)** Patients were first subdivided according to the presence or absence of anti-epitopes, i.e. non-spreaders (anti-Cys R) vs. spreaders (anti-CysRC1, anti-CysRC7, anti-CysRC1C7). **(c)** In a second analysis, non spreaders and spreaders were split for the positivity for anti-SOD2, anti- αENO and anti-AR1 antibodies.

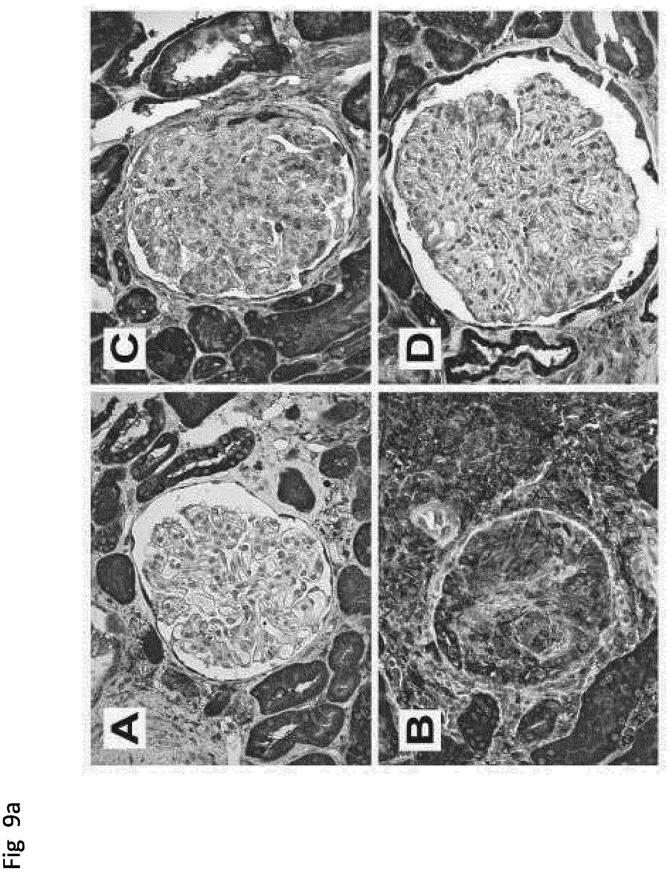

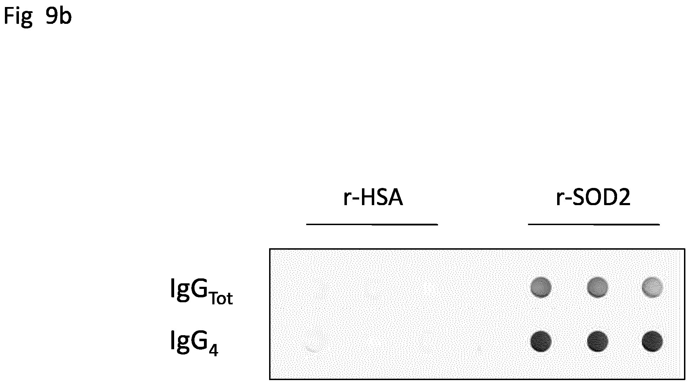

**Figure 9****. SOD2. a-**Expression of SOD2 (immunohistochemistry) and presence of anti-SOD2 IgG4 in glomeruli (laser capture and dot-blot) of the same patients who presented at To medium and low levels of circulating anti-SOD2 igG4. A-normal glumerulus; B-Lupus Erythematosus with class IV lesions; C-D glomeruli from two patients with high and median levels of circulating SOD2. b-dot-blot analysis for anti-SOD2 IgG4 in three microdissected glomeruli of patients with MN (two corresponded to pt C-D)

### DETAILED DESCRIPTION OF THE INVENTION

The present invention relates to an *in vitro* method for predicting clinical outcome of a patient with membranous nephropathy (MN) who has received a pathologic diagnosis, an *in vitro* method for monitoring a therapy against nephritis in subjects affected by MN.

### In vitro method for the Prediction cinical outcome of proteinuria and renal function in patients with MN

The herein described *in vitro* method allows predicting the clinical outcome in a subject affected by MN. With the term 'clinical outcome ' it is here indicated the remission of proteinuria and normal renal function after one year of follow up.

Under Membranous Nephropathy (herein designated even under the abbreviation MN from the English term Membranous nephropathy) in the present invention, according to what indicated in the medical/scientific literature, one relates to a chronical disease with autoimmune nature characterized by a typical nephropathy characterized by proteinuria, nephrotic syndrome and pathologic lesions characterized by sub-epithelial deposition of antibodies, particularly of IgG4 isotype. MN is considered the basic model for any autoimmune glomerulonephritis ("primary MN") or occurs as a manifestation of systemic conditions, including cancer, infections, reactivity to drugs or lupus erythematosus ("secondary MN"){Glassock, 2010 #80;Ronco, 2015 #460;Lefaucheur, 2006 #90}. It is of note that the natural history of the disease may vary in relation of unknown factors: about 25-30% of MN patients undergo spontaneous remission, others respond to treatments while 25% show progression to end stage renal disease in spite of any therapeutic approaches{Fervenza, 2008 #458;Polanco, 2010 #459}.

If not timely identified and treated, also potentially responsive cases of MN may evolve towards the terminal chronical renal insufficiency so as to require extracorporeal dialysis and renal transplant as necessary treatments for survival. Under Membranous Nephropathy, (herein designated even with the abbreviation MN from the English term) in the present invention, according to the clinical/medical practice, a clinico-pathology condition is meant characterized by an alteration of the structure and of the renal function that is characterized by the following typical immunopathological features: a- the deposit in the renal glomeruli of antibodies (IgG, IgA, IgM) and more specifically of IgG4 as well as the complement (C3, C4); b- the presence of sub-epithelial deposits of immune complexes sometimes associated with focal or diffused proliferative lesions.

The *in vitro* methods described in the present invention aim at determining, preferably in the peripheral blood, antibodies which have been found in the kidney of the patients affected by MN and, therefore, herein characterized for the first time as predictors of the disease. The dosage allows predicting a poor clinical outcome of renal lesions early in subjects affected by MN in absence or in presence even of slight symptoms which can be associated to the disease.

In particular, it is possible carrying out the prediction of clinical outcome at the time of diagnosis and such method could be used in the follow up of patients with MN already as from the beginning of the general clinical symptoms and in absence of renal involvement by preceding the beginning of the full blown picture of the disease.

In particular, the herein described method comprises a passage a) of determining the concentration of IgG4 antibodies of at least one of the antigens chosen from the group: SOD2 and/or αenolase, in a biological sample of a subject affected or potentially affected by MN. In a preferred embodiment of the invention, the antibody thereof the concentration is determined is an anti-SOD2 and/or anti-aenolase IgG4 antibody; b) of determining the concentration of antibodies versus epitopes of PLA2r

The determination of the concentration of the above antibodies starting from any biological material can be performed according to any one of the methods considered by the person skilled in the art suitable to this purpose. Such methods are widely known in literature and described in details in most part of the laboratory manuals, therefore it does not result to be necessary to examine them closely on this occasion.

By purely way of example, the concentration of the antibodies anti-SOD2 and/or αenolase of IgG4 type in a biological sample can be determined by means of methods such as ELISA (enzyme-linked Immunoabsorbent assay), western-blot, RIA (radioimmunoassay), proteic arrays, dot-blot or mass spectrometry and in any case more generally with all techniques used in the clinical chemistry allowing to detect a reaction between an antigen and an antibody by detecting with quantitative methods the occurred reaction thereof. The above techniques exploit the possibility of making the antibody under evaluation to interact with the specific antigen and then to detect the quantity of antibody linked to specific antibodies versus the human IgG4; the last reaction is detected, for example, by means of horseradish peroxidase (HRP) conjugated to the anti-human IgG4 antibodies. The horseradish peroxidase, by catalysing the oxidation of a substrate thanks to the hydrogen peroxide, generates a coloured, fluorescent or luminescent product, which can be measured by means of spectrophotometer. It is obvious that any detecting system linked to the anti-IgG4 antibody can be used. In particular, the determining methods preferably will be immunological methods.

In an embodiment of the invention, the determination of the antibody anti-SOD2 and/or αenolase, of IgG4 isotype can be performed with a monoclonal primary antibody, such as for example those commercialized by Invitrogen (Clone: HP6014-InVitrogen Corporation, Camarillo, CA) or however polyclonal antibodies able to link to the above antigens.

By following the techniques known to the person skilled in the art for detecting antigens and the antigen-antibody complex, then in a biological sample the levels of IgG4 antibodies, specific for SOD2 and/or αenolase, will be determined. In particular, the (primary) anti-SOD2 and/or αenolase antibodies will recognize in a specific way an epitope existing in the described proteins and which in turn can be then recognized by a suitable secondary antibody (anti-human IgG4), directed towards the primary antibody. Such secondary antibody could be marked, for example, with any fluorochrom commonly used in marking secondary antibodies such as, for example, fluorescent substances (by way of illustration:FITC, Cy3, Cy5, Alexa 488, PEe) or enzymes or substances which can be detected by means of enzymatic cytochemistry (for example horseradish peroxidase) to allow then the detection of the primary antibody and then of the antibody anti-SOD2 and/or anti-aenolase of IgG4 type.

In exemplifying way, the determination of the antibody anti-SOD2 and/or anti-aenolase of IgG4 isotype able to recognize the herein described antigens, can be made even by using a proteic array constituted by a solid support whereon different reagents are deposited (in time and logical series) in order to obtain the reaction between antigen and specific antibody (method known as dot-blot). In exemplifying way in case of anti-SOD and anti-aenolase IgG4 antibodies, for example, the specific antigen that is SOD2 or αenolase is deposited in known concentrations ("spotted", in technical slang) on solid supports (paper, resins, other supports); in a second passage on the support a complex mixture is deposited, such as for example a cell lysate, a blood or serum sample in order to allow the reaction between antigen and specific antibody existing in the mixture; in a third passage the IgG4 reacting with SOD2 and/or with αenolase by means of anti-human IgG4 antibodies are displayed.

The determination of the concentration of such antibodies of IgG4 class can be performed, according to what herein described, starting from any biological sample which reasonably comprises the antibody which one wants to detect.

In an embodiment of the invention the biological sample is selected from the group comprising: blood, serum, renal tissue biopsy. Advantageously in a preferred embodiment of the invention, the biological sample is represented by a sample of blood or serum of the examined subject.

According to the type of sample and of the type of technique chosen for determining the concentration of the antibodies of the invention, the person skilled in the art will be able, based upon the common laboratory knowledge, to arrange what is necessary (such as for example sample preparation/processing) in order to perform the herein-described in vitro method.

After determining the concentration of IgG4 antibodies versus at least one of the antigens (anti-SOD2, anti-aenolase), the subject method provides a passage wherein the concentration determined in the biological sample of the examined subject is compared with a control value. Under control value a reference value related to concentration of IgG4 antibodies for the specific antigen in samples obtained by subjects not having MN is meant. Such controls can be chosen for example from the group of: healthy subjects, subjects affected by nephritis with aetiology different from MN, such as for example, focal segmental glomerulosclerosis.

In a preferred embodiment of the invention, such control value is obtained from samples of healthy subjects or subjects affected by other glomerulonephritis .

As it will be clear to the person skilled in the art, the control value preferably will be the average value of the concentration of IgG4 antibodies able to recognize the herein indicated antigens calculated based upon a group of healthy subjects or subjects affected by other rheumatic and/or renal pathology.

In particular, according to the herein described in vitro method for the prediction of MN in a subject affected or potentially affected by MN who has anti-PLA2r and anti-epitope antibodies, an increase in the concentration of anti-SOD2 and/or anti-aenolase IgG4 with respect to the control value indicates a potentially worse outcome in respect to MN patients who do not present anti-SOD2 and/or anti- anti-aenolase IgG4. The antibody quantity for example can be determined as arbitrary unit of optical density expressed as [O.D. unit] corresponding to a determined chemiluminescence signal intensity unit, by way of example, with VersaDoc system equipped with QuantyOne software (Bio-Rad) and it is then calculated and expressed as concentration of the antibody in mg per I (mg/l) of serum by making reference to a standard curve with known content of IgG4.

With respect to the passages during the laboratory determination and to the comparison we remand to what already has been indicated in previous parts of this document.

### In vitro monitoring method

The present invention relates even to a method for predicting and monitoring the clinical outcome of MN in patients who have been tested at the beginning of the disease.

For the definition of MN see what previously said for the *in vitro* method for the above prediction.

In particular, the monitoring *in vitro* method comprises the passage of a) determining the concentration of IgG4 antibodies versus at least one of the antigens selected from the group: SOD2 and/or αenolase, in at least a first and at least a second biological sample of a subject affected or potentially affected by MN, wherein said samples are obtained in different time; b) of determining the concentration of antibodies versus PLA2r and anti-PLA2r epitopes

Under the term "monitoring" herein the control of the clinical outcome of the pathological status or pathological condition, in this case MN, of a patient in time is meant. Therefore, under the term monitoring, in the present invention, performing one or more determinations of the concentration of anti-SOD2 and/or anti-aenolase IgG4 antibodies in a subject under examination in a certain time interval is designated. By purely way of example and not with limitative purposes, a monitoring can have as aim the evaluation of responsiveness of a subject to a determined therapy, preferably against MN. In such optics, then, the determination of anti-SOD2 and/or anti-aenolase IgG4 antibodies could be for example performed on samples obtained from a given subject affected by MN first, during and/or after a general time interval or therapeuticapproach. In this case, the possible improvement or worsening or stationarity of the pathological state in the subject method in terms of variation in the concentration of anti-SOD2 and/or anti-aenolase IgG4 antibodies in the considered time interval corresponds to the possible benefit, or not, of the therapy thereto the subject is submitted.

Even in this case the determination of the concentration of IgG4 antibodies anti-SOD2 and/or anti-aenolase igG4 can be performed starting from any biological sample which reasonably comprises the antibody which one wants to detect. For more detailed information about the biological sample one refers to the analogous section existing in the above in vitro method for the prediction and/or the diagnose of MN. In particular, the biological samples compared for the monitoring, for example, a first and a second sample can be independently chosen from the group comprising: blood, serum, renal tissue biopsy.

Furthermore, the technical aspects related to the methods for determining the concentration of anti-SOD2 and/or anti-aenolase IgG4 antibody useful to the purpose of the monitoring method, are to be considered analogous to those already described above for the method for predicting and/or diagnosing MN.

The determination of the concentration of the antibody of interest to the monitoring purpose should be performed at least in a first biological sample and in at least a second biological sample of a subject obtained at different time, then for example respectively at a time t=0 and t>0. The subject under examination, in particular, can be both a subject submitted to a therapy against MN and a subject monitored in time without being necessarily submitted to any type of therapy. In other terms, then, said at least first biological sample obtained at time t=0 can be a sample, for example, before starting the therapy itself, and instead in a subject not submitted to therapy, acquired at a general time t=0. Differently, the second biological sample can be acquired at one or more time intervals starting from said time t=0, therefore defined as time t>0, which by pure way of example can be intervals of hours, days, or months, for example every 15 days. In each case the person skilled in the art, depending upon the subject type and upon the gravity of the pathology, will be able, based upon his/her knowledge, to identify the most suitable time interval to the purpose of herein described monitoring method. Preferably, the first and second sample are respectively obtained before starting a therapy against MN and during and/or after said therapy. Subsequently, the comparison between the concentration of the anti-SOD2 and/or anti-aenolase IgG4 antibody obtained in said first and said at least second sample will provide information about the progression of the pathological sate of the patient.

The variation in the concentration of the antibody of interest, for example anti-SOD2 and/or anti-aenolase IgG4, is then the instrument allowing to the clinician to evaluate the effectiveness, or not, of the chosen therapeutic strategy. In a particular embodiment an increase in the concentration of IgG4 antibody in the second sample with respect to the first sample indicates the progression of MN in a subject affected or potentially affected by MN.

Alternatively, a decrease in the concentration of IgG4 antibodies in the second sample with respect to the first sample designates a non-progression of MN in a subject affected or potentially affected by MN.

In particular, the type of therapy to be monitored is a general therapy against MN. In particular, the therapy can include treatment with inhibitors of the Angiotensin Converting Enzyme (ACE), high-dosage cortisone, cyclophosphamide, cyclosporine and rituximab.

### Kit for predicting and/or for monitoring a therapy against Membranous Nephropathy.

A subject of the present invention is also a kit for predicting outcome in terms of proteinuria and renal function or for monitoring a therapy against MN in subjects suffering or potentially suffering from MN.

In particular, such kit comprises at least an aliquot of one or more reagents for the determination of IgG4 antibodies versus at least one of the antigens selected from the group: SOD2 and/or αenolase, in a biological sample of the subject under examination and at least an aliquot of one positive control comprising IgG4 antibodies versus at least one of the antigens selected from the group: SOD2 and/or αenolase,

In a preferred embodiment of the present invention, said one or more reagents are needed for determining the concentration of anti-SOD2 and/or anti-aenolase IgG4 antibodies.

Preferably, the kit according to the invention could include at least a primary polyclonal or monoclonal antibody able to recognize such antigens (to be used as standard curve) and, optionally, anti-human IgG4 antibodies. The kit could even include one or more aliquots of a marked or not marked secondary antibody, said secondary antibody being, obviously, specific for the primary antibody used in the standard curve. Then, if the primary antibody is implemented in mouse, the secondary will be anti-mouse, if implemented in rabbit it will be anti-rabbit and so on. Alternatively, the kit could include a plate thereon there are the dosage theme antigen to allow performing a dosage ELISA.

The kit could additionally include negative controls and/or positive controls. In particular, by purely way of example and not with limitative purpose, the kit can include as positive control the sera of patients (patients could be different) with documented high serum levels of anti-SOD2 and/or anti-αenolase IgG4. As positive controls even aliquots of the proteins of interest can be provided, that is SOD2 and/or αenolase. As negative control, for example the serum of a pool of patients with known glomerulonephritis can be used, wherein the antibodies of interest cannot be dosed. In this case negative sera of patients with membraneous glomerulo nephritis will be used. Furthermore, the kit can include suitable reagents and means for the procedure of determining the concentration of anti-SOD2 and/or anti-aenolase IgG4 antibodies such as aliquots of buffer solutions, sterile water or other reagents commonly used for detecting, for example, the primary antibody-secondary antibody complex.

The following examples and experimental results have the purpose of designating the ways for implementing the present invention, however without limiting the same.

In a preferred embodiment of the kit, as well as of the method described above, the determination of the concentration of antibodies of IgG4 type able to recognize the antigens chosen from the group: SOD2 and/or αenolase is carried out by means of dot-blot or ELISA.

*Dot-blot.* The analysis is performed by using an apparatus for dot-blot of Bio-Rad (Hercules, CA,USA) and it is based upon the immobilization of the antigen under dosage (SOD2, αenolase) on membrane of nitro-cellulose. The membrane pretreated with TBS is fixed to the apparatus before charging a constant quantity of antigen in TBS (100 ng). The antigen first of all is left in contact for 24 h and then additionally linked to the nitro-cellulose with negative pressure, created with vacuum. The membrane with linked antigens is then saturated in TBS-T (TBS-5% albumin-0.05% gr/v,Tween20 v/v). The serum under dosage dil 1:50 in TBS-T is applied at this point in the wells and left incubating for 6 hours at the environment temperature and then overnight at 4 C; at the end the membrane is washed 3 times in TBS-T. At this point the presence of IgG4 is detected by incubating at the environment temperature, with anti-human IgG4 antibody (Clone: HP6014-InVitrogen Corporation, Camarillo, CA) diluted 1:2000 in TBS-T marked with horseradish peroxidase (HPR). After 3 washings wish TBST-T the reaction is developed in chemiluminescence (SuperSignal, West Pico, Chemiluminescent, Thermo scientific, Rockford, USA. A standard curve using IgG4 antibody with different dilutions is prepared to evaluate the reaction linearity and establishing the reading range.

**ELISA** for determining anti-SOD2 anti- αenolase the antigen is placed in the single wells of a plate with 96 wells (MaxiPrep plate 96 wells) and incubated at room temperature for 5 h and then at 4°C overnight with serum. Aliquots (200*µ*l) of locking solution (5% BSA in PBS and 0.05% Tween20) are added in each well, before adding the serum (100*µ*l) diluted 1:50 in PBS-T (PBS - Tween20 0.05% v/v-BSA 1% gr/v) which is incubated for 4 hours at room temperature and then overnight at 4C°. After 3 washings in PBS-T, anti-human IgG4 antibodies (Clone: HP6014-InVitrogen Corporation, Camarillo, CA) diluted 1:3000 in TBS-T and marked with horseradish peroxidase (HPR) are added. The development of the reaction to peroxidase is obtained by adding 100 *µ*l of substrate TMB/H₂O₂ (10:1) and by incubating for variable time depending upon the colorimetric reaction (Mx 30 minutes). Such colorimetric reaction is then locked by adding 100 *µ*l of solution 0.45 M of H₂SO_{4.} and read within 30 min. The absorbance is then read at 450 nm in suitable reader,multiplate reader, iMark(BioRad,Hercules, CA, USA). A standard curve using IgG2 antibody at different dilutions is prepared for evaluating the reaction linearity and establishing the reading range.

### MATERIALS AND METHODS

**Patients.** Three-hundred-thirty-two patients were recruited at the time of diagnosis immediately after having a pathology diagnosis of membranous glomerulonephritis and were followed on clinical ground for at least 12 months; a part had successive serum and urinary controls up to 36 months. Demographic characteristics are reported in Table 2. Criteria for enrollment were (1) a biopsy-based diagnosis of MN, (2) a normal complement profile (3) negative tests for ANA, nDNA, and ANCA and cryoglobulins and the absence of viral markers (hepatitis B surface antigen and HIV). Kidney histology was evaluated on Dubosq-Bresil solution-fixed tissues embedded in paraffin, sectioned, and stained with hematoxylin/eosin, Masson trichrome, silver methenamine, and periodic acid-Schiff. Specimens for EM were fixed in Karnovsky solution and processed using standard methods. IgG, IgA, IgM, C1q, C3, and fibrinogen depositions were detected by direct IF staining on frozensections. Blood from the cohort described was obtained the day before renal biopsy and after centrifugation at 1.55 _ g for 15minutes at 4°C, was immediately stored at -80°C. At that time no therapy were started yet.

Therapy consisted in cytotoxic drugs in 107 cases, rituximab in 40 while 36 patients received both approaches; 55 patients were treated with symptomatic (ACEi) alone and 69 received no treatment in the 12 months of the follow up. Clinical activity of the disease during the follow-up was evaluated on the basis of proteinuria levels in response to treatment, which we defined as complete remission (proteinuria < 0.3 gr/day)and partial remission (proteinuria 0.3-3.5 gr/day or reduced by at least 50% from baseline.Analysis of proteinuria was done with a turbidimetric assay. Renal function was calculated with the CKD-EPI creatinine 2009 equation

**Normal subjects.** Serum was obtained from 50 normal controls recruited at the same institutions that participated to the sample recruitment. They consisted of normal blood donors who had at least one normal urinalysis and normal clinical tests in the prior 6 months (Table 1).

### Analysis of Circulating antibodies

*Antibodies.* Purified rabbit antiserum against human SOD2 was purchased from Upstate Biotechnologies (Charlottesville, VA); recombinant human Aldose Reductase (H00000231), recombinant human SOD2 (H00002023) and recombinant human □Enolase were obtained from AbNova (Tapei, Taiwan); anti-human synaptopodin (clone G1D4-IgG1) were obtained from Progen Biotechnik, (Heidelberg, Germany) and mouse mAb anti-human C5b-9 (clone aE11-IgG2a) were from Dako).

*IgG4.* Purified mouse mAb to human IgG4 (clone HP6025, IgG1-K) was purchased from Invitrogen (Camarillo, CA).

*Secondary Antibodies.* Affinity-purified FITC F(ab_)2 donkey anti- human IgG, affinity-purified FITC and Texas Red-conjugated donkey anti-goat IgG, and donkey anti-rabbit IgG and FITC affinity-purified donkey antimouse IgG all were purchased from Jackson Immunoresearch (WestGrove, PA). For IEM, donkey anti-goat or anti-rabbit IgG conjugated with 12 nm of colloidal gold particles (Jackson Immunoresearch) was used.

*Normal limits.* ROC curves tested specificity and sensitivity and gave the limit of positivity (Cut Off value) for each antibody calculated as the value that minimizes the geometric distance from 100% sensitivity and 100% specificity on the ROC curves.{Zweig, 1993 #476;Zweig, 1993 #477}For anti-hPLA2r IgGs levels the limit of positivity given by the manufacturer was utilized.{Dahnrich, 2013 #481}

*Anti-human PLA2r.* For anti-hPLA2r we utilized the commercial ELISA test of Euroimmun (Medizinische Labordiagnostika AG, Lubecca, Germany). Results are expressed RU form a standard calibrator with known anti-PLA2r IgGs levels (2-1.500 RU/ml)

*Anti- PLA2R epitopes. The* titer of circulating autoantibodies (IgG4) versus versus epitopes of human PLA2r we utilized recombinant and soluble form of CysR, CTLD1 and CTLD6-7 with HA-tag domains containing PLA2R epitopes. CysR and CTLD6-7 were produced in HEK293 cells and CTLD1 in E. Coli. Plates were coated with anti-HA antibody (Sigma-Aldrich) diluted at 1:5000 in 20 mM Tris pH 8.0 (100 µl/well) at 4°C/overnight or with purified rabbit PLAR1 (100 ng/well) or mouse PLA2R (50 ng/well). Plates were blocked for 2h with Seramun Block (Seramun Diagnostica). In the well coated with anti-HA antibodies, cell medium from HEK293 cells transfected with soluble forms of PLA2R (10-100 µl/well) or purified E.Coli HA-CTLD1 fusion protein (50 ng/well) were then added and incubated for 1 h. Plates were washed and patients' sera diluted at 1:100 in PBS/0.1% dry milk were added in duplicate (100 µl/well) . After 2 h incubation at room temperature on a plate shaker, plates were washed and anti-IgG4-horse radish peroxidase conjugate (Southern Biotech) diluted 1:7500 in SeramunStab ST plus was added (100 µl/well) and incubated for 1h at room temperature on a plate shaker. After four washes, tetramethylbenzidine was added, and the reaction were developed for 15 min and then stop with HCI 1.2N. The plates were read at 450 nm. Results were in all cases expressed as chemiluminescence OD arbitrary units that corresponds to one unit of signal intensity of chemiluminescence detected by VersaDoc and computed with Quanty One software (Bio-Rad). The limit of positivity of each antibody was the value that minimized the geometric distance from 100% sensitivity and 100% specificity on the ROC curves{Zweig, 1993 #476;Zweig, 1993 #477}.

*Anti-AR and Anti-SOD2 and anti*-αenolase *IgG4.* The titer of circulating anti-SOD2, anti-AR and anti-alphaenolase IgG4 in sera of patients with MN normal control subjects were determined with dot blot utilizing recombinant proteins as fixed antigen for anti-. The Dot-Blot essay was done with a Bio-Dot apparatus (Bio-Rad, Hercules, CA, USA) following the instruction manual with minor modifications. All samples and the calibrator serum were diluted with Tris buffered saline (TBS) pH 7.4 (1:100). A calibration curve was prepared by keeping constant the amount of recombinant protein and increasing dilution of specific antibody from 1:500 to 1:32,000. Sera were diluted in the same buffer (1:100) to achieve the desired range of levels, obtained after testing several conditions.

Accordingly, the nitrocellulose membrane was pre-wetted in TBS and placed on a sheet of Whatman 3 mm filter paper embedded with the same buffer. Constant amounts of protein (300 ng) were placed in the 80 □l of TBS. After removal of the air bubbles between the two sheets by gentle pressure, the sample template was placed on the nitrocellulose membrane and a vacuum was applied for a few minutes, to fill up the 96 sample wells with 50 mL using a multi-channel pipette. The vacuum was applied until all the samples were adsorbed. The same operation was repeated five times with 150 uL of buffer each, to wash out the non-adsorbed sample. The nitrocellulose was then gently removed and saturated with 5% w/v bovine serum albumin (BSA) in TBS. Sera were then left for six hours at room temperature; at the end the membrane was washedsix times in 0.15% v/v Tween-TBS. Incubation with HPR-anti human IgG1-3-4 0.5 □g/mL in 1% w/v BSA in TBS was performed for two hours room temperature. The membrane was then washed four times, 15 min each, with TBS-T prior to developing the immuno reaction with SuperSignal West Pico Chemiluminescent substrate (Thermo scientific, Rockford, IL, USA). Chemiluminescence was detected by VersaDoc and computed with QuantyOne software (Bio-Rad) and given as relative optical density [O.D. unit] that corresponds to one unit of signal intensity. Results are expressed as mg/l IgG4 calculated form a standard curve with known levels of IgG4 (0.002-2mg/l). The limit of positivity of each antibody was the value that minimized the geometric distance from 100% sensitivity and 100% specificity on the ROC curves{Zweig, 1993 #476;Zweig, 1993 #477}.

### Immunohistochemistry for SOD2.

Renal histological sections (3-µm) were obtained from the original paraffin tissue blocks and used for immunohistochemical analyses. A primary antibody anti-Superoxide dismutase 2 (SOD 2, Abcam, rif. ab16956, Cambridge, UK; dilution 1:400) was immunohistochemically tested.The histological sections were dewaxed in xylene, rehydrated through graded alcohol, and, after quenching endogenous peroxidase with a 3% H₂O₂ water solution, incubated with a protein block (Ready to Use DakoCytomation Biotin Blocking System, DakoCytomation California Inc, Carpinteria, CA, USA). The slides were incubated for 30 minutes at room temperature, and the reactions were revealed using streptavidin-biotin-peroxidase complex (K0675, LSAB 2 System, DakoCytomation) followed by a solution of 3-3'-diaminobenzidine tetrahydrochloride. In the end, they were counterstained with Mayer hematoxylin, mounted, and coverslipped. The negative control procedure consisted of the omission of the primary antibody.

### Laser Capture Microdissection and determination of Antibodies from Renal Biopsy Tissue

Cryostatic sections (4 micron) of kidney tissue specimens were placed on metal frame slides with thermoplastic membrane (Molecular Machines & Industries AG; Glattburg, Zurich, Switzerland), stained, and dehydrated using an Arcturus HistoGene, LCM Frozen Section Staining Kit (Arcturus Bioscience, Mountain View, CA) according to the manufacturer's instructions. Air-dried sections were then viewed with the NIKON ECLIPSE-TE 2000 inverted microscope (Nikon-Instruments, Sesto Fiorentino, Italy). Glomeruli were identified and isolated with the Molecular Machines & Industries Cellcut LMD system by focal melting of the membrane through laser activation. The Molecular Machines & Industries Cell cut Laser Capture Microdissection system is equipped with a solid-state ultraviolet laser that guarantees precise cutting without damaging the tissue. High precision-stage and CCD camera allow identification, documentation, and dissection of multiple regions of interest from the same tissue specimen. For each specimen, a total of 25 to 30 glomeruli were microdissected and removed sequentially in separate isolation cap (Nikon Instruments)with special adhesive material in the lid. After visual control of the completeness of dissection, captured tissue was immersed in denaturation buffer and used for proteomic analysis. Sections of human kidney derived from non carcinomatous portions of kidneys removed for renal carcinoma were used as negative control. Igs were recovered from glomeruli by means of acid elution as described previously.34 Briefly, after washings with PBS (0.01 M, pH 7.2) groups of 20 glomeruli were incubated overnight with 0.02 M citrate buffer (pH 3.2) at 4°C in a humid atmosphere. After removal of citrate buffer, 0.4 M NaOH was added to achieve a pH of 7.2.

### Statistical Analysis

Statistical analysis were performed with R software. Auto-antibodies levels were expressed as median and interquartile range. The differences in serum concentration of all auto-antibodieswithinpatient groups were analyzed using non-parametric U Mann-Whitney test for unpaired sample; Spearman's analysis was used to evaluate correlations among circulating levels. The receiver operation characteristic (ROC) analysis was used to evaluate the discrimination capacity of each auto-antibodies. The best cut-offs were selected as the values that minimized the geometric distance from 100% sensitivity and 100% specificity on the ROC curves{Zweig, 1993 #476;Zweig, 1993 #477}. Statistically significance were defined when the area under the curves (AUC) were > 0.5.

Odds ratios (OR) and 95% confidence intervals (CI) were tested to determine the association between clinical parameters and protenuria and Fisher's exact test was used to determine the statistically significance. Kaplan Meier survival curves for remission events were computed according to the level of protenuria (protenuria< 0.3 gr/die; between 0.3 and 3.5; >3.5). Differences in outcomes were estimated using the log-rank (Mantel-Cox) test. P-values ≤ 0.05 were considered significant.

### EXAMPLES

**1.Patients.** Overall 332 patients with iMN were recruited in the study and tested for the presence of circulating antibodies that are considered characteristic of iMN (i.e. anti- human and anti-orthologs PLA2r, anti-epitopes, anti-THSD7A anti-SOD2, anti-AR and anti-Eno). The whole panel was analysed in 292 (supplement Figure 1). Men were more represented than women in this sample (M/F 229/103) (supplement Table 1) and males had an increased risk to remain proteinuric at 12 months from diagnosis(supplement Figure 2). Sixty-two percent of patients received cytotoxic agents, rituximab or both; 17% received cyclosporine and 62% received ACE inhibitors as unique treatment or in association with cytotoxic agents or Rituximab; 21% were not treated at all. Most patients (191) had proteinuria>3.5 gr/day at diagnosis (To) that was associated with a higher risk of not reaching complete remission (supplement Figure 2). **2. Anti-hPLA2r, Anti-PLA2r epitopes.** We determined circulating anti-PLA2r levels using an ELISAs Levels, normal limits, variability and other quantitative data are reported in Table 1. IgG4 versus different epitopes of the molecule (i.e. anti-CysR, anti-C1, anti-C6C7) were also tested with specific ELISA and, on the basis of positiveness , patients were divided in 4 sub-groups: (1) anti-CysR+; (2) antiCysRC1+; (3) anti-CysRC7+ and (4) anti-CysRC1C7+. As expected, circulating levels were very high for the whole panel (Figure 1 a-f). **3. Anti-THSD7A.** Antibodies against THSD7A were determined by a ELISA (Figure 1g), immunofluorescence and then confirmed by western-blot. Only 8 cases matching the three techniques were considered positive (Figure 2d); all were negative for anti-hPLA2r. A few THSD7A positive cases were also positive for anti-SOD2, anti-AR and anti-eno IgG4 (Figure 2e). **4. Anti-SOD2, Anti-AR, Anti-eno.** *Circulating levels.* Positivity for circulating anti-SOD2, anti-AR or anti-eno IgG4 was found in 88 (30%), 89 (30%) and 122 patients (42%) respectively. Forty-eight patients were positive for only one of these antibodies and specifically 10 for anti-SOD2, 22 for anti-AR and 16 for anti-eno. Mixed positivity for anti-podocyte antibodies and anti-PLA2r (here including anti-epitopes) predominated in this cohort of patients. However, serum levels of anti-SOD2, anti-AR and anti-eno IgG4 did not correlate each other and did not correlate with anti-PLA2r levels (Figure 2e).

*Renal expression.* SOD2 expression was evaluated by immune-histochemistry (Figure 3a) in 6 patients with variable circulating levels of anti-SOD2 antibodies (3 with high and 3 with medium-levels). Three normal donor kidneys at transplantation and 3 renal biopsies from patients with lupus nephritis (class 3-4) were studied for comparison. Glomeruli were, in parallel, micro-dissected from biopsies of patients with high circulating anti-SOD2 levels for determining anti-SOD2 IgG4 in micro-eluates. SOD2 was undetectable in normal kidneys, comparably expressed in high SOD2 patients and very high in lupus glomeruli. Anti-SOD2 IgG4 were very high in all the 3 micro-dissected biopsies utilized for analysis (Figure 3b). **5-Risk assessment and survival.** *Proteinuria.* Clinical activity of the disease during the follow-up was evaluated on the basis of proteinuria levels in response to treatment, which was defined according to shared guidelines{Thompson, 2015 #198} as complete (proteinuria <0.3 gr/day) and partial remission (proteinuria 0.3-3.5 gr/day or reduced by at least 50% from baseline). Persistence of proteinuria >3.5 gr/day was defined as no-remission. The probability of reaching complete or partial remission associated with the presence of antibody in the serum (i.e. Ab+ vs. Ab-) was calculated in the whole cohort or in subgroups defined on the basis of proteinuria at To (i.e., proteinuria above or below 3.5 gr/day). In the whole cohort, serum positivity of the following antibodies was associated with the risk of failure to achieve complete remission: anti-hPLA2r (OR 2.87 [95%CI 1.54-5.34]),(OR 2.79 [95%Cl 1.37-5.66]), anti-CysRC1C7 epitopes (2.67 [95%CI 1.03-6.94]) and anti-SOD2 (OR 2.43 [95%CI 1.17-5.04]) The same strong association was found when patients were considered on the basis of treatment, that means the presence of any of the antibodies above still maintained a worse prognosis in spite they had received a treatment. In this case, patients negative for all antibodies had a high probability to achieve remission of proteinuria after treatment (OR 0.27 [95%CI 0.11-0.66]). When patients were subdivided on the basis of proteinuria at To, only anti-SOD (OR 3.58[95%CI 1.29-9.95]) were associated with an even higher risk of failure to achieve complete remission. The combination of anti-SOD2 and anti-hPLA2r identified patients with the highest 5,33 (1,16-24,55). Risks associated with the presence of any antibody and considering partial remission of proteinuria as outcome were in all cases non significant.

Kaplan Meier curves were analyzed considering proteinuria as above (complete, partial and no remission) and patients were subdivided in positive or negative to each antibody (Figure 5a-f). Analysis relative to the positivity versus anti-PLA2r epitopes was restricted to patients positive for anti-hPLA2r who were, however, the majority. Considering the first outcome (complete remission) patients positive for anti-hPLA2r anti-CysRC6C7 and anti-SOD2 had a worse outcome at T₁₂. Combining positivity for anti-hPLA2r and anti-SOD2 gave the best fitting allowing to identify those patients negative to both who presented the best outcome (60% reached remission at T₁₂). Considering partial or no remission as outcome, no significant fitting were found. Combining positivity for anti-epitopes and anti-SOD2, anti-alpha enolase almost the 100% of those patients who do not reduced proteinuria after 1 year was identified that is much more of those who were only anti-epitope positive (Figure 8a-c) **6-Levels of antibodies during follow-up.** Serum levels of all antibodies were determined at To and at two time intervals (T₆, T₁₂) (Figure 1a-i). The median levels of each antibody were reduced at T₆ and T₁₂ for some categories (i.e. anti-PLA2r-epitopes, anti-SOD2, anti-AR and anti-alpha enolase IgG4). Levels of the remaining antibodies (anti-hPLA2r, anti-TSHD7A) decreased without reaching significant values (Figure 1b-e).

*In conclusions, considering the cohort of patients we studied, 4 subgroups of MN patients presenting a different composition of circulating antibodies were identified: a- a small cohort* of 8 *patients was uniquely positive to THSD7A; b-the great majority of patients were positive to anti-hPLA2r IgGs* (63%) *and 65 (22%) were uniquely positive to these antibodies; c- a significant cohort had a mixed positivity (i.e. anti-hPLA2r, plus anti-AR, anti-SOD2 and anti-alpha enolase); d-less than 5% had no antibodies. Therefore, a part of the data here presented are confirmatory of previous work. New results that open to clinical applications and actually represent an advancement in interpreting mechanism of MN are the finding on prediction of clinical outcome at T12 of patients on the basis of circulating levels of autoantibodies. The main findings are that anti-hPLA2r, anti-PLA2r epitopes and anti-SOD2 and anti-alpha enolase levels can predict outcome and prediction still persists when treatments were considered; in this case it is of great clinical interest that we could show that the absence of any antibody identify patients that have a very low risk of bad outcome. While the results on anti-PLA2r (of every kind, including epitopes) although new are covered by a patent, the results on the additive value of anti-SOD2 antibodies to anti-PLA2r epitopes antibodies (that have been discovered in my laboratory) are completely new and should be covered by a new patent. A second main finding of the study is that we consider renal function after 12 months of therapy, the levels in serum of anti-alpha enolaselgG4 at T₀ predicted a CPK-EPI <60 ml min 1.73 m² after one year.*

**Table 1. Clinical details relative to the cohort of patients with MN enrolled in this study.**

| | **iMN** | Controls |
|---|---|---|
| **n** | 332 | 50 |
| Male gender n (%) | 229 (69) | 35 (69) |
| | | |
| Age (years) | 61 (11-87) | 52 (18-60) |
| | | |
| Histological stage n (%) | | |
| **I** | 56 (24%) | |
| **II** | 107 (46%) | |
| III | 52 (22%) | |
| IV | 19 (8%) | |
| | | |
| | | |
| Serum creatinine (mg/dl) | 1.1 (0.3-7) | 0.6 (0.4-1) |
| | | |
| Proteinuria (g/day) | | 0.1 (0.05-0.15) |
| at diagnosis | 5.6 (0.5-25) | |
| after 12 months | 1.7 (0.1-33) | - |
| Serum albumin (g/dl) | | 4.2 (4-4.5) |
| at diagnosis | 2.5 (1.0-4.2) | |
| after 12 months | 3.6 (1.5-4.8) | - |
| | | |
| ACE-i / ARB therapy | 62% | |
| | | |
| Cytotoxic | 45% | |
| Cyclosporine A | 17% | |
| Rituximab | 17% | |
| None | 21% | |

## Claims

1. An *in vitro* method for the prediction of the clinical outcome in terms of proteinuria in a patient affected by Membranous Nephropathy comprising the following steps:
a) determining the concentration of IgG4 antibodies versus SOD2 and alpha enolase, in a biological sample of said subject;
b) comparing said concentrations in said biological sample with a control value,
wherein an increase of the concentration of said antibodies with respect to said control value indicates a high risk of no developing a remission of the disease after one year.

2. The method according to claim 1 further comprising a step of determining the concentration of IgG4 antibodies anti-PLA2r.

3. The method according to claim 1 or 2 further comprising a step of determining the concentration of IgG4 antibodies anti-PLA2r epitopes.

4. The method according to any one of the claims 1 to 3, wherein said biological sample is selected from the group comprising: blood, serum, renal tissue biopsy.

5. The method according to any one of the claims 1 to 4, wherein said control value is obtained from biological samples of healthy subjects or subjects affected by other glomerulonephritis.

6. An *in vitro* method for monitoring the progression of membranous nephropathy in a patient affected by the disease comprising the following steps:
a) determining the concentration of IgG4 antibodies versus at least one of the antigens selected from the group: SOD2 and αenolase
in at least a first and at least a second biological sample of said subject, said at least a first and a second samples obtained at different times,
b) comparing said concentration obtained for said first and second sample, wherein an increase in the concentration of said antibodies in said second sample with respect to said first sample indicates the absence of remission of membranous nephropathy in said subject.

7. The *in vitro* method according to claim 6 wherein said at least a first and second sample are respectively obtained before starting a therapy and during and/or after said therapy.

8. The *in vitro* method according to claims 6 and 7, wherein an increase in the concentration of said antibodies in said second sample with respect to said first sample indicates the absence of remission of membranous nephropathy in said subject.

9. The *in vitro* method according to any one of the claims from 6 to 8, wherein a decrease in the concentration of said antibodies in said second sample with respect to said first sample designates a non-progression of membranous nephropathy in said subject.

10. The *in vitro* method according to any one of claims from 1 to 9 wherein said determination is carried out by ELISA (enzyme-linked Immunoabsorbent assay), dot-blot, western-blot, RIA (radioimmunoassay), immunochemistry or mass spectrometry.

11. The *in vitro* method according to any one of claims from 1 to 10 further comprising a step of determining the concentration of IgG4 antibodies anti-PLA2r, anti-PLA2r epitopes, anti-AR1 and/or anti-THSD7A.

12. The *in vitro* method according to any one of claims from 1 to 11 wherein an increase of the concentration of said antibodies with respect to said control value at the start of the disease or when it is diagnosed indicates a high risk of achieving a decrease of renal function under the limit of 60ml/min/1,73m2 after one year.

13. A kit for the prediction the clinical outcome of membranous nephropathy and/or for monitoring a therapy in subjects suffering or potentially suffering from the disease comprising:
- at least an aliquot of one or more reagents for the determination of IgG4 antibodies versus SOD2 and an aliquot of one or more reagents for the determination of IgG4 antibodies versus αenolase in a biological sample of said subject and
- at least an aliquot of one positive control comprising IgG4 antibodies versus SOD2 and/or αenolase.

14. The kit according to claim 13 further comprising an aliquot of one or more reagents for the determination of IgG4 antibodies versus at least one of the antigens selected from the list that includes anti-PLA2r, anti-PLA2r epitopes, anti-AR1 and/or anti-THSD7A.

15. The kit according to claim 13 or 14, wherein:
- said one or more reagents comprise at least a primary polyclonal or monoclonal antibody capable of recognizing at least one of said antigens and, optionally, anti-IgG4 antibodies and/or
- said one aliquot of positive control comprises at least total extracts of mesangial cells and/or podocytes obtained from patients with membranous nephritis.
